(19) Europäisches Patentamt / European Patent Office / Office européen des brevets

(11) **EP 2 999 409 B1**

(12) # EUROPÄISCHE PATENTSCHRIFT

(45) Veröffentlichungstag und Bekanntmachung des Hinweises auf die Patenterteilung:
**13.09.2017 Patentblatt 2017/37**

(51) Int Cl.:
*A61B 6/00* (2006.01)    *A61B 6/06* (2006.01)
*G21K 1/06* (2006.01)

(21) Anmeldenummer: **14728479.8**

(22) Anmeldetag: **22.05.2014**

(86) Internationale Anmeldenummer:
**PCT/EP2014/060501**

(87) Internationale Veröffentlichungsnummer:
**WO 2014/187885 (27.11.2014 Gazette 2014/48)**

(54) **PHASENKONTRAST-RÖNTGENBILDGEBUNGSVORRICHTUNG**

PHASE-CONTRAST X-RAY IMAGING DEVICE

DISPOSITIF D'IMAGERIE RADIOGRAPHIQUE PAR CONTRASTE DE PHASE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.05.2013 DE 102013008925**

(43) Veröffentlichungstag der Anmeldung:
**30.03.2016 Patentblatt 2016/13**

(73) Patentinhaber: **Siemens Healthcare GmbH**
**91052 Erlangen (DE)**

(72) Erfinder: **PREUSCHE, Oliver**
**91052 Erlangen (DE)**

(56) Entgegenhaltungen:
**WO-A1-2013/004574    US-B1- 6 428 939**

- **ZHOU S A ET AL: "Development of phase-contrast X-ray imaging techniques and potential medical applications", PHYSICA MEDICA, ACTA MEDICA EDIZIONI E CONGRESSI, ROME, IT, Bd. 24, Nr. 3, 2. September 2008 (2008-09-02), Seiten 129-148, XP024341442, ISSN: 1120-1797, DOI: 10.1016/J.EJMP.2008.05.006 [gefunden am 2008-07-07]**

EP 2 999 409 B1

**Beschreibung**

Die Erfindung betrifft eine Phasenkontrast-Röntgenbildgebungsvorrichtung, also eine Röntgenvorrichtung für eine Phasenkontrastbildgebung, insbesondere für eine Phasenkontrastbildgebung im Medizinbereich, umfassend eine Röntgenstrahlungsquelle zur Generierung eines Röntgenstrahlungsfeldes und einen Röntgendetektor mit einer eindimensionalen oder zweidimensionalen Anordnung von Pixeln.

Die Wechselwirkung von elektromagnetischer Strahlung im Allgemeinen, und Röntgenstrahlung im Speziellen, mit einem Medium wird üblicherweise durch Angabe eines komplexen Brechungsindex beschrieben. Realteil und Imaginärteil des Brechungsindexes sind dabei jeweils abhängig von der materiellen Zusammensetzung des Mediums, dem der komplexe Brechungsindex zugeordnet ist. Während der Imaginärteil die Absorption der elektromagnetischen Strahlung in dem Medium wiedergibt, beschreibt der Realteil des Brechungsindex die materialabhängige Phasengeschwindigkeit, und damit die Brechung der elektromagnetischen Strahlung.

Aktuell eingesetzte Röntgenbildgebungsvorrichtungen dienen meist ausschließlich zur Darstellung eines Absorptionskontrastes und detektieren dementsprechend die materialabhängige Strahlungsabsorption in einem zu untersuchenden Objekt, wobei die Intensität der durch das Objekt transmittierten Röntgenstrahlung ortsaufgelöst aufgezeichnet wird.

Weniger verbreitet ist derzeit noch die Ausnutzung der von dem Objekt verursachten Brechung und der damit einhergehenden materialabhängigen Phasenverschiebung zum Zwecke der Darstellung eines Phasenkontrastes für die Bildgebung. Entsprechende Verfahren und Vorrichtungen werden jedoch bereits entwickelt.

Zur messtechnischen Erfassung der Phasenverschiebung wird hierbei typischerweise ein Talbot-Lau-Interferometer eingesetzt, wie es beispielsweise in "X-ray phase imaging with a grating interferometer, T. Weitkamp at al., 8. August 2005/ Vol. 13, No. 16/OPTICS EXPRESS" beschrieben ist.

Bei einem herkömmlichen Talbot-Lau-Interferometer sind entlang einer optischen Achse eine Röntgenstrahlungsquelle, ein Kohärenzgitter $G_0$, ein Phasengitter (oder Beugungsgitter) $G_1$, ein Analysegitter (oder Absorptionsgitter) $G_2$ und ein aus einer Vielzahl von Pixeln aufgebauter Röntgendetektor angeordnet. Das Kohärenzgitter $G_0$ dient dabei zur Sicherstellung einer ausreichenden räumlichen Kohärenz der Röntgenstrahlungsquelle. Dementsprechend kann das Kohärenzgitter $G_0$ im Falle einer annähernd punktförmigen Röntgenstrahlungsquelle entfallen. Mit Hilfe des Phasengitters $G_1$, welches typischerweise eine gleichmäßige gestreifte Struktur aufweist, wird ein Interferenzmuster erzeugt, dessen Intensitätsverteilung mittels des Röntgendetektors detektiert wird.

Die Periode dieses Interferenzmusters ist dabei typischerweise deutlich kleiner als die Größe der Pixel des Röntgendetektors, so dass eine direkte Erfassung des Interferenzmusters mit dem Röntgendetektor nicht möglich ist. Um dennoch das Interferenzmuster vermessen zu können, ist dem Röntgendetektor daher üblicherweise das Analysegitter (oder Absorptionsgitter) $G_2$ vorgeschaltet, mit dessen Hilfe das Interferenzmuster durch eine räumlich-periodische Ausblendung von Röntgenstrahlung abgetastet werden kann. Hierzu wird das Analysegitter $G_2$ in einer Ebene senkrecht zur optischen Achse und der Struktur des Interferenzmusters verschoben. Alternativ zu dem Analysegitter $G_2$ können auch das Kohärenzgitters $G_0$ oder das Phasengitter $G_1$ verschoben werden.

Für die Phasenkontrastbildgebung wird das zu untersuchendes Objekt zwischen der Röntgenstrahlungsquelle (und dem gegebenenfalls vorhandenen Kohärenzgitter) einerseits und dem Phasengitter $G_1$ andererseits positioniert. Alternativ hierzu kann das Objekt auch zwischen dem Phasengitter $G_1$ und dem Analysegitter $G_2$ positioniert werden. In beiden Fällen verursacht das Objekt eine ortsabhängig variierende Phasenverschiebung der Röntgenstrahlung oder eine Änderung der Richtung der Wellenfronten der Röntgenstrahlung, die das durch das Phasengitter $G_1$ erzeugte Interferenzmuster, also die Intensitätsverteilung der Röntgenstrahlung, messbar verändert. Das veränderte Interferenzmuster wird dann auf die oben beschriebene Weise mittels des Röntgendetektors detektiert und aus der gemessenen Intensitätsverteilung des Interferenzmusters wird schließlich auf die ortsabhängige Phasenverschiebung zurückgerechnet, wobei aus der Phase oder aus der Phasenverschiebung die Bildinformation unmittelbar gewonnen wird. Alternativ kann die Bildinformation auch aus der Dichte (d.h. der integrierten Phase) oder der Winkelstreuung (Dunkelfeld) ermitteln werden. Ferner wird mitunter das Phasenkontrastbild mit einem gleichzeitig gewonnenen Absorptionskontrastbild verrechnet, um das Bildrauschen zu reduzieren.

Der gewünschte Vorteil der Phasenkontrast-Röntgenbildgebung besteht dabei darin, dass sich Strukturen im sogenannten Weichteilgewebe (insbesondere Gewebe, Wasser und Körperfette) im Phasenkontrast in der Regel stärker voneinander abheben als im Absorptionskontrast. Dabei wird das Auflösevermögen einer entsprechenden Phasenkontrast-Röntgenbildgebungsvorrichtung im Wesentlichen bestimmt durch die Eigenschaften des eingesetzten Talbot-Lau-Interferometers und insbesondere durch dessen geometrische Abmessungen. Hierbei lässt sich das Auflösevermögen oder die Empfindlichkeit zum Beispiel dadurch erhöhen, dass auf eine höhere Talbot-Ordnung ausgewichen wird und dementsprechend der Abstand zwischen dem Phasengitter und dem Analysegitter beispielsweise um den Faktor 3 vergrößert wird. Allerdings werden hierdurch die Abmessungen des gesamten Aufbaus vergrößert und zudem wir die nutzbare Bandbreite des Spektrums der elektromagnetischen Strahlung begrenzt. Alternativ oder ergänzend hierzu lässt sich das Auflösevermögen auch durch die Verwendung von Gittern mit feineren Strukturen vergrößern, jedoch besteht hier das Problem, dass die Herstellung feinerer Strukturen mit einem größeren technischen Aufwand verbunden ist.

Aus WO 2013/004574 A1 ist eine Phasenkontrast-Röntgenbildgebungsvorrichtung zum Einsatz in der Mammographie offenbart. In diesem Zusammenhang ist offenbart, dass die aufzunehmende Brust möglichst entfernt von dem Detektor der Vorrichtung zu platzieren ist, um den Phasenkontrast in dem aufgenommenen Röntgenbild zu erhöhen.

Ausgehend hiervon liegt der Erfindung die Aufgabe zugrunde, die Phasenkontrast-Röntgenbildgebung zu verbessern.

Diese Aufgabe wird erfindungsgemäß gelöst durch eine Phasenkontrast-Röntgenbildgebungsvorrichtung mit den Merkmalen des Anspruchs 1. Vorteilhafte und teils für sich gesehen erfinderische Ausgestaltungsformen und Weiterentwicklungen der Erfindung sind in den Unteransprüchen und der nachfolgenden Beschreibung dargelegt.

Eine entsprechende Phasenkontrast-Röntgenbildgebungsvorrichtung ist dabei insbesondere für den Medizinbereich ausgelegt und umfasst eine Röntgenstrahlungsquelle zur Generierung eines Röntgenstrahlungsfeldes und einen Röntgendetektor mit einer eindimensionalen oder zweidimensionalen Anordnung von Pixeln. Dabei ist zwischen der Röntgenstrahlungsquelle und dem Röntgendetektor ein Phasenkontrast-Differenzverstärker positioniert und mit dessen Hilfe werden im Betrieb räumliche Phasenunterschiede im Röntgenstrahlungsfeld verstärkt.

Es werden also insbesondere lokale, durch ein Untersuchungsobjekt hervorgerufene Phasenverschiebungen gezielt verstärkt, so dass hierdurch deutlichere Änderungen in einem Interferenzmuster auftreten, die tendenziell leichter messtechnisch erfasst werden können. Auf diese Weise ist dann ein Aufbau realisiert, bei dem die Empfindlichkeit im Vergleich zu einem klassischen Talbot-Lau-Interferometer erhöht ist, ohne dass hierfür die Gitterperiode eines eingesetzten Gitters verkleinert wurde und ohne dass die Einbaulänge vergrößert wurde. Somit wird quasi die Empfindlichkeit von den rein geometrischen Größenverhältnissen entkoppelt.

Als Röntgenstrahlungsquelle wird dabei zweckdienlicherweise eine schmalbandige Röntgenstrahlungsquelle eingesetzt, also beispielsweise eine Röntgenröhre mit einem nachgeschalteten Filter, der Röntgenstrahlung in einem sehr schmalen Frequenzband passieren lässt, oder auch eine insbesondere lasergetriebene Synchrotron-Strahlungsquelle, wie beispielsweise das kompakte CXS. Dabei lässt sich prinzipiell unterschiedlich harte Röntgenstrahlung nutzen und dementsprechend wird zweckdienlicherweise eine an den jeweiligen Anwendungszweck angepasste Röntgenstrahlung eingesetzt (z.B. Anodenspannung der Röntgenröhre 25 kVp für Mammographie oder 100 kVp für Computertomographie).

Prinzipiell ist dieser Aufbau und insbesondere der Phasenkontrast-Differenzverstärker, oder zumindest das dahinter stehende Prinzip, auch auf Anwendungen mit sichtbarem Licht oder elektromagnetischer Strahlung benachbarter Wellenlängen (IR, UV) übertragbar und beispielsweise für einen Shack-Hartmann-Sensor nutzbar.

Weiter ist der Phasenkontrast-Differenzverstärker bevorzugt derart ausgestaltet, dass unbeeinflusste Röntgenstrahlung, also insbesondere Röntgenstrahlung, die nicht durch ein zu untersuchendes Objekt eine Richtungsänderung der Wellenfront erfahren hat, eine einheitliche Phasenverschiebung durch den Phasenkontrast-Differenzverstärker erfährt. Die Phasenverschiebung durch den Phasenkontrast-Differenzverstärker ist also zum Beispiel zunächst unabhängig von der Eintrittsposition der unbeeinflussten Röntgenstrahlung am Phasenkontrast-Differenzverstärker.

Zudem wird mittels des Phasenkontrast-Differenzverstärkers vorzugsweise auch das für die interferometrische Messmethode benötigte Interferenzmuster erzeugt und dementsprechend ersetzt der Phasenkontrast-Differenzverstärker in diesem Fall das Phasengitter $G_1$ eines klassischen Talbot-Lau-Interferometers. Die einheitliche Phasenverschiebung durch den Phasenkontrast-Differenzverstärker erfolgt dann wie bei einem Phasengitter $G_1$ eines klassischen Talbot-Lau-Interferometers. Bei diesem wird typischerweise ein rechteckiges binäres Profil aus Stegen und Zwischenräumen genutzt, um ein streifenförmiges Interferenzmuster zu erzeugen, wobei die Phase der Röntgenstrahlung an den Stegen um n (bzw. $\lambda/2$) verschoben wird ("PI-Gitter"). Der Phasenkontrast-Differenzverstärker umfasst zwei Brechungsgitter, die in einer Strahlungseinfallrichtung gesehen nacheinander angeordnet sind, wobei der Phasenkontrast-Differenzverstärker bevorzugt zwei gleiche Brechungsgitter aufweist. Dementsprechend wird gemäß einer vorteilhaften Ausgestaltung das Phasengitter $G_1$ eines klassischen Talbot-Lau-Interferometers durch zwei gleiche Brechungsgitter ersetzt, mit deren Hilfe einerseits das Interferenzmuster erzeugt mit und mit deren Hilfe zudem lokale, durch ein Untersuchungsobjekt hervorgerufene Phasenunterschiede verstärkt werden. Der Idee, das das Interferenzmuster erzeugende, beugende oder brechende Element in einem Talbot-Lau-Interferometer, also das Phasengitter $G_1$, durch zwei lichtbrechende Elemente, also zwei Brechungsgitter $G_A$ und $G_B$, zu ersetzen, liegen dabei die nachfolgend dargelegten Überlegungen zugrunde.

Beim klassischen Talbot-Lau-Interferometer werden Änderungen von Richtungen der Wellenfronten elektromagnetischer Strahlung, also quasi Phasengradienten, anhand mikroskopisch kleiner Intensitätsänderungen erkannt. Dabei wird mittels des Phasengitters $G_1$ ein streifenförmiges Interferenzmuster dadurch erzeugt, dass jeder Steg des Phasengitters $G_1$ die Phase der auftreffenden Röntgenstrahlung üblicherweise um eine halbe Wellenlänge verschiebt (alternativ erfolgt eine Verschiebung um eine viertel Wellenlänge). Infolgedessen ist in einem bestimmten (ungeraden) Vielfachen des sogenannten "Talbot"-Abstandes hinter dem Phasengitter $G_1$ ein Interferenzmuster aus schmalen Streifen feststellbar, wobei an dieser Position dann das Analysegitter $G_1$ positioniert wird.

Bei einer vereinfachten Betrachtung verhält sich das Intensitätsmuster dabei so, wie ein vom Phasengitter $G_1$ erzeugter Schatten. Bei einer entsprechenden Ausrichtung des Analysegitters $G_2$ relativ zum streifenförmigen Intensitätsmuster wird eine maximale oder eine minimale Intensität $I_{max}$ bzw. $I_{min}$ das Analysegitter $G_2$ passieren, wobei aus der mittels des Röntgendetektors messtechnisch erfassten Intensität wiederum auf die Phasen $\Phi = 0$ bzw. $\Phi \pm 1 \pi$ zurückgeschlossen wird.

Das typischerweise zusätzlich eingesetzte Kohärenzgitter oder Absorptionsgitter $G_0$ dient hierbei lediglich dazu, die Auswirkungen der Ausdehnung der Röntgenstrahlungsquelle, meist eine Röntgenröhre, zu kompensieren. Dieses wird direkt an der Röntgenröhre platziert und stellt sicher, dass jeder seiner Schlitze als Strahlungsquelle dasselbe Streifenmuster auf das Analysegitter $G_2$ projiziert (ohne $G_0$ würden sich viele Streifenmuster überlappen und eine einheitliche Intensität an $G_2$ bewirken). Bei Verwendung einer Röntgenstrahlungsquelle, die in sehr guter Näherung ebene Wellen generiert (Synchrotron), oder bei Röntgenstrahlungsquellen, die in guter Näherung als punktförmige Quellen (Mikrofokus) angesehen werden können, kann das Absorptionsgitter $G_0$ entfallen.

Da die Phasenkontrast-Bildgebungsvorrichtung insbesondere für den Einsatz im Medizinbereich vorgesehen ist, ist weiter von besonderer Bedeutung, wie die Absorption der Röntgenstrahlung durch das Analysegitter G2 die benötigte Röntgendosis beeinflusst.

Hierbei ist die Transmission T hinter einem zu untersuchenden Objekt, also insbesondere hinter einem Patienten, durch das Intensitätsverhältnis $T = I / I_0$ bestimmt, wobei $I_0$ die durchschnittliche Intensität ohne $G_1$ und $G_2$ ist. Das Rauschen (repräsentiert durch die Standardabweichung $\sigma_\varphi$) ist dabei proportional zu $(\Delta t\, I_0)^{-2}$ (wie in Monte-Carlo-Simulationen), d.h. um das Rauschen zu halbieren, wird die vierfache Dosis $\Delta t\, I_0$ benötigt.

Bei differentiellem Phasenkontrast (sowie auch bei Dunkelfeldaufnahmen) ergibt sich eine proportionale Abhängigkeit des Rauschens $\sigma_\varphi$ von der Streifen-Phase $\varphi$ :

$$\sigma_\varphi^{\,2} \;\propto\; 1/(\Delta t \;\; I_0 \;\; V^2 \;\; T)$$

Dabei ist V die (Streifen-)Sichtbarkeit oder Visibilität mit:

$$V = (I_{max} - I_{min}) \;/\; (I_{max} - I_{min})$$

Die Größen $I_{max}$ und Imin bezeichnen hierbei die maximalen/minimalen Intensitäten abhängig von der x-Position eines verschiebbaren $G_0$, wobei Sichtbarkeit V und die Transmission T jeweils in den Grenzen 0-100% variieren können.

Um das Rauschen auf einem bestimmten Wert zu halten, muss also $\Delta t\, I_0\, V^2\, T$ unverändert bleiben. Es kann eine Effizienz $\eta$ der Optik hinter dem Patienten definiert werden als:

$$\eta_\varphi \;=\; V^2 \;\; T$$

Ein Ziel der vorliegenden Anmeldung ist, $\eta_\varphi$ zu optimieren, d.h., eine Dosisminimierung bei gegebenem $\sigma_\varphi$ (im Unterschied zu $\sigma_{\Phi'}$ oder $\sigma_\Phi$) zu erreichen, wobei $\Phi$ die Phase der tatsächlichen Wellenfront und $\Phi'$ deren räumliche Änderung $\Phi' = \partial\Phi/\partial x$ bezeichnen. $\Phi'$ ist dabei gegeben durch:

$$\Phi' \;=\; \partial\Phi/\partial x \;=\; \varphi \;\; p_2 \;/\; (\lambda \;\; d_{12})$$

wobei $p_2$ die Gitterperiode von $G_2$ ist, was der doppelten Stegbreite $s_2$ des Gitters entspricht, und wobei $d_{12}$ der Abstand zwischen den Gittern $G_1$ und $G_2$ ist.

Mit der Definition der Empfindlichkeit S als $S = d_{12} / p_2$ gilt bei gegebener Design-Wellenlänge $\lambda = \lambda_D$:

$$\sigma_{\Phi'} \;\propto\; \sigma_\varphi \;/\; S$$

Je höher also die Empfindlichkeit, desto geringer ist somit das Rauschen.

Aus:

$$\sigma_{\Phi'}^{\,2} \;\propto\; \sigma_\varphi^{\,2} \;/\; S^2 \;\propto\; 1/(\Delta t \;\; I_0 S^2 V^2 T)$$

folgt wie oben bei vorgegebenem Rauschen $\sigma_{\Phi'}$ dass die Dosis $\Delta t \cdot I_0$ proportional ist mit $\eta_{\Phi'}$, wobei:

$$\eta \;=\; \eta_{\Phi'} \;=\; S^2 V^2$$

wobei $\eta = \eta_{\Phi'}$ die Effizienz der Messung hinter dem Patienten ist.

Bei bisherigen Ausführungen ist $\eta$ typischerweise zu klein, insbesondere bei hohen Photonenenergien (z.B.: 100 kVp Röhrenspannung und entsprechend beispielsweise 65 keV Design-Energie bzw. 19 pm Design-Wellenlänge der Photonen) ergibt sich somit bislang für Phasenkontrastbilder in der Regel eine höhere Strahlenbelastung für einen Patienten als bei der Absorptionsbildgebung. Dabei wird als Grund in der Fachliteratur eine niedrige Sichtbarkeit V angegeben.

Da jedoch sehr viele Messwerte relativ klein ausfallen, würde eine Erhöhung der Empfindlichkeit des Aufbaus für die meisten Messwerte auch eine geringere Sichtbarkeit kompensieren. Müssen dennoch Messwerte erfasst werden, die dadurch aus dem Messbereich herausfallen, so besteht prinzipiell die Möglichkeit im Rahmen einer Nachfolgemessung mit geringerer Empfindlichkeit und deutlich geringerer Strahlendosis (d.h. hohem Rauschen) diese Stellen zu identifizieren und die entsprechenden Messwerte zu korrigieren (wenn auch mit höherem Rauschen).

Wie zuvor dargelegt, ist die Empfindlichkeit des Aufbaus dabei proportional zur Gitterperiode $p_2$ oder auch proportional zur Wurzel des Gitterabstandes $d_{12}$. Insbesondere den Abstand zwischen den beiden Gittern $G_1$ und $G_2$, also $d_{12}$, will man dabei jedoch nicht beliebig vergrößern. Günstiger wäre es, eine höhere Empfindlichkeit zu realisieren, ohne die Gitterperioden wesentlich zu reduzieren, da dies die Sichtbarkeit insbesondere bei hohen Photonenenergien reduziert. (bei niedrigeren Photonenenergien z.B. 30 keV, ist es dagegen möglich, einfach auf eine höhere Talbot-Ordnung auszuweichen. Man vergrößert also den Abstand $d_{12}$ zum Beispiel um den Faktor 3 und erhält erneut ein sichtbares Interferenzmuster mit höherer Empfindlichkeit.) Es stellt sich somit die Frage, welche anderen Möglichkeiten es gibt, die Empfindlichkeit weiter zu erhöhen.

Betrachtet man nun ein klassisches Talbot-Lau-Interferometer, so wird mit diesem letztlich der Winkel des einfallenden Lichtes oder der Röntgenstrahlung, also die Richtung der Wellenfronten, ermittelt. Wird nun beispielsweise das Phasengitter $G_1$ um einen kleinen Winkel um eine y-Achse gedreht, die durch den Mittelpunkt von $G_1$ verläuft und parallel zu den Stegen von $G_1$ ist, so ändert sich im Prinzip nichts an der Position des Interferenzmusters, da die Drehung die sichtbare Periode praktisch nicht beeinflusst und die Abweichungen auf einer z-Achse, entlang derer die Gitter des Talbot-Lau-Interferometers angeordnet sind, nicht ins Gewicht fallen, weil Längen entlang der z-Achse um ein Vielfaches größer sind als die Gitterperioden entlang einer x-Richtung, die senkrecht zur y-Achse einerseits und zur z-Achse andererseits ist.

Wird nun in dieser Situation die Röntgenstrahlungsquelle zusammen mit $G_0$ und $G_1$ gedreht, so dass $G_1$ wieder die ursprüngliche Position einnimmt, so ändert sich die Ausrichtung des Interferenzmusters bezüglich $G_2$, welches nicht mitbewegt wurde, und zwar genau entsprechend der Drehung der einfallenden Strahlung, also der Drehung der Röntgenstrahlungsquelle zusammen mit $G_0$ um den Mittelpunkt von $G_1$. Das bedeutet, die Ausrichtung zwischen dem Interferenzmuster und $G_2$ gibt exakt die Richtung der in $G_1$ einfallenden Strahlung wieder, gerade so als wäre das Interferenzmuster lediglich ein Schattenwurf von $G_1$ und alles passiert rein geometrisch.

Dabei wird für das Phasengitter $G_1$ typischerweise ein rechteckiges binäres Profil verwendet, bei dem quasi streifenförmige Stege und streifenförmige Freiräume alternierend nebeneinander angeordnet sind, wobei die Phase der elektromagnetischen Strahlung an den Stegen um n oder $\lambda/2$ verschoben wird. Dabei wird in der Regel von einer Beleuchtung mit ebenen Wellen ausgegangen und dementsprechend gilt für den Abstand $d_{12\parallel}$ zwischen dem Phasengitter $G_1$ und dem Analysegitter $G_2$:

$$d_{12\parallel} = p_1^2/(8\lambda)$$

Bei einer inkohärenten Quelle mit kugelförmiger Ausbreitung (Röntgenröhre) verändert sich der Abstand dagegen entsprechend der Linsengleichung:

$$1/f = 1/g + 1/b$$

mit der Brennweite f = $d_{12\parallel}$, der Gegenstandsweite g=$d_{01}$ und der Bildweite b=$d_{12}$ (da Weglängenunterschiede indirekt proportional zu Abständen entlang der optischen Achse sind und da die Linsengleichung diese indirekte Proportionalität wiedergibt).

Alternativ lassen sich auch höhere ungeradzahlige Talbot-Abstände, wie beispielsweise dem Dreifachen, dem Fünffachen oder Siebenfachen, für die Positionierung des Analysegitters $G_2$ nutzen (dies verkleinert jedoch die Gitterperioden, sofern die Einbaulänge $d_{12}$ beibehalten wird, weswegen bei höheren Photonenenergien üblicherweise davon abgesehen wird).

Nachfolgend wird jedoch ein Phasengitter $G_1$ betrachtet, welches die Phase um $\pi/2$ bzw. $\lambda/4$ verschiebt ("PI-Halbe-Gitter") und die halbe Periode des zuvor genannten PI-Gitters besitzt. Da hier $d_{12\parallel} = p_1^2/(2\lambda)$ ist, kann in einem Aufbau ein "PI-Gitter" direkt gegen "PI-Halbe-Gitter" (der halben Periode) getauscht werden, ohne dass die Abstände entlang der optischen Achse oder die Gitterperioden ($p_0$ oder $p_2$) angepasst werden müssen.

Dies gilt zwar auch für höhere ungeradzahlige Talbot-Abstände, jedoch besitzt das "PI-Halbe-Gitter" bei Nutzung von elektromagnetischer Strahlung mit zu breitem Frequenzspektrum die unangenehme Eigenschaft, dass die Interferenz-Streifenmuster je nach Wellenlänge teilweise um eine halbe Periode verschoben sind, so dass die Sichtbarkeit hierdurch reduziert ist. Auch aus diesem Grund werden in der nachfolgenden Betrachtung keine höheren Talbot-Abstände genutzt.

Es zeigt sich nun, dass bei einem "PI-Halbe-Gitter" $G_1$ maximale Sichtbarkeit dann gegeben ist, wenn dieses elektromagnetische Strahlung einer Design-Wellenlänge $\lambda_D$ um genau ein Viertel der Wellenlänge (oder 90°) verschiebt. Erfolgt hingegen eine geringere Verschiebung (z.B. nur um f* $\lambda/4$ mit f* aus dem Bereich [-100 %,+ 100 %]), so reduziert sich die Sichtbarkeit des Interferenzmusters sinusförmig, das heißt, die Sichtbarkeit sinkt auf etwa sin(f* $\pi/2$) der ursprünglichen Sicht-

barkeit. Insbesondere bei kleinen Werten von f* ist der Kontrast also nahezu linear zu f*, wobei auch ein negativer Kontrast möglich ist, das heißt, das Bereiche höherer und niedrigerer Intensität im Streifenmuster die Positionen tauschen (bei einem "PI-Halbe-Gitter" betragen die Weglängen-Unterschiede zwischen einem direkten Strahl durch zwei korrespondierende Streifenmitten und einem durch eine benachbarte Streifenmitte ein Viertel der Design-Wellenlänge. Damit reicht eine viertel Wellenlänge (also $\pi/2$) an Phasenhub aus, um in einem Fall die Weglängen-Unterschiede zu kompensieren und im anderen Fall Auslöschung zu erreichen. Diese Situation ist symmetrisch, wird zum Beispiel statt bei vormals Streifen einer geraden Nummerierung nun bei ungerade nummerierten Streifen ein Phasenhub von PI-Halbe eingebaut, so kompensieren sich die Unterschiede nun dort an $G_2$, wo sie sich vorher ausgelöscht hatten und umgekehrt).

Ausgehend von diesen Betrachtungen wird nun ein neuer Ansatz verfolgt, gemäß dem der Winkel der einfallenden elektromagnetischen Strahlung oder die Richtung der Wellenfronten, repräsentiert durch die Steigung $\Phi' = \partial\Phi/\partial x$ der Phase $\Phi$ der einfallenden elektromagnetischen Strahlung quasi übersetzt wird in einen periodischen rechteckigen Phasenhub der Größe $f* \lambda/4$, so dass $f* \sim \Phi'$, wobei dann für $f*$ bzw. $\Phi'$ die Sichtbarkeit des Interferenzmusters proportional zu $f*$ bzw. $\Phi'$ ist. Die Messung der Sichtbarkeit erlaubt dabei die Ermittelung der Steigung der Phase und somit der Richtung der Wellenfronten.

Ist dann die einfallende elektromagnetische Strahlung über einen gewissen kleinen Winkelbereich gestreut, so mitteln sich die Kontrastwerte für die einzelnen Winkel gegenseitig aus. Das heißt, dass Dunkelfeld-Signal muss nicht mehr sichtbar sein, positiv gesehen stört es aber die Messung der Phase über die Sichtbarkeit auch nicht. Nimmt der Streuwinkelbereich jedoch zu, so wird er irgendwann natürlich die Sichtbarkeit reduzieren.

Ist die Empfindlichkeit hoch, wobei sich $f*$ stark mit $\Phi'$ ändert, dann wird sich die Position der Streifen des Interferenzmusters praktisch nicht ändern bei kleinen $\Phi'$, und es reicht eine Messung des Kontrastes $(I_2 - I_1) / (I_2 + I_1)$ (z.B. zwei Intensitäts-Messungen $I_1$ und $I_2$, wobei $G_2$ um eine halbe Periode $s_2 = p_2$ zwischen den beiden Messungen verschoben wird).

Im Falle einer Nutzung des Phasenkontrast-Differenzverstärkers in einem Computertomographen ließe sich in diesem Zusammenhang die Methode aus der Anmeldung US 2012/0041679 A1 anwenden. Darin ist beschrieben, dass nach einer halben Umdrehung der Gantry des Computertomographen die Ablenkung des Lichts das umgekehrte Vorzeichen hat, sowie auch der Kontrast im Falle der zuvor beschriebenen Messmethode, so dass die helleren und dunkleren Bereiche gegeneinander vertauscht sind. Hierdurch würde sich eine Verschiebung irgendwelcher Gitter erübrigen.

Die gewünschte Übersetzung der Steigung der Phase in einen entsprechenden Phasenhub wird nun mit Hilfe zweier Brechungsgitter $G_A$ und $G_B$ realisiert, die dann das Phasengitter G1 eines klassischen Talbot-Lau-Interferometers ersetzen. Die beiden Brechungsgitter $G_A$ und $G_B$ sind dabei bevorzugt gleichartig ausgestaltet und nach Art eines Linsengitters $G_L$ ausgeführt, wie es in der WO 2013/160 153 A1 beschrieben ist. Genau genommen stellen die Brechungsgitter $G_A$ und $G_B$ einen einfachen Grenzfall des Linsengitters $G_L$ dar. Die Brechungsgitter $G_A$ und $G_B$ weisen hierbei, genau wie ein Linsengitter $G_L$, eine Transversalfläche auf, die durch eine x-Achse und eine hierzu senkrechte y-Achse aufgespannt wird, und die im Wesentlichen (d.h. exakt oder zumindest näherungsweise) quer zu einer Strahlungseinfallrichtung auszurichten ist. Die vorgesehene Strahlungseinfallrichtung definiert hierbei eine z-Achse des Brechungsgitters, die in der vorgesehenen Einbauposition des Brechungsgitters insbesondere parallel zu einer optischen Achse der Röntgenbildgebungsvorrichtung ausgerichtet ist. Die Transversalfläche kann hierbei innerhalb des von dem Brechungsgitters eingenommenen Raumvolumens grundsätzlich an beliebiger z-Position (d.h. Position entlang der z-Achse) definiert werden. Lediglich beispielhaft wird im Folgenden angenommen, dass die Transversalfläche durch die "vordere" Stirnfläche des Brechungsgitters gebildet ist, über die die Strahlung in das Brechungsgitter einfällt.

Die vorstehend eingeführten Achsen spannen ein karthesisches Koordinatensystem auf. Die durch die Orientierung der x-, y- und z-Achse definierten Raumrichtungen sind dabei nachfolgend als (positive) x-, y- bzw. z-Richtung bezeichnet. Die jeweils entgegengesetzten Raumrichtungen sind als (negative) als x-, y- bzw. z-Richtung bezeichnet. Positionen auf der x-, y- und z-Achse sind als x-, y, bzw. z-Positionen bezeichnet.

Entsprechend der gewünschten Eigenschaft des Linsengitter $G_L$, benachbarte verschiedenartige Strukturen des Interferenzmusters in unterschiedliche Fokusse (d.h. Fokalpunkte oder Fokallinien) zu brechen, ist die Transversalfläche eines Linsengitter $G_L$ in jeweils in y-Richtung langgestreckte Brechungsstreifen gegliedert, die in x-Richtung parallel nebeneinander aufgereiht sind. Benachbarte Brechungsstreifen unterscheiden sich hierbei dadurch, dass sie hinsichtlich der Brechungseigenschaften des im Bereich dieses Brechungsstreifens jeweils angeordneten Gittermaterials stets auf verschiedene Fokusse ausgerichtet sind. Mit anderen Worten ist das Material des Linsengitters $G_L$, das entlang der z-Achse über und/oder unter einem Brechungsstreifen angeordnet ist, derart ausgestaltet, dass es Strahlung zumindest einer bestimmten Designwellenlänge in einen bestimmten Fokus bricht, während das Material des Linsengitters $G_L$, das entlang der z-Achse über und/oder unter einem benachbarten Brechungsstreifen angeordnet ist, die Strahlung in einen anderen Fokus bricht. Bei der Transversalfläche und den darauf gebildeten Brechungsstreifen handelt es dabei sich um mathematischabstrakte Strukturen.

Die strahlungsbrechende Wirkung des Linsengitters $G_L$ wird durch eine Mehrzahl von Brechungsstegen aus einem optisch vergleichsweise dünnen Basismaterial (also einem Feststoff mit für Röntgenstrahlen vergleichsweise niedrigem Realteil des Brechungsindex) erzeugt, wobei diese Brechungsstege alternierend mit optisch vergleichsweise dichten Zwischenräumen angeordnet sind. Bedingt durch die trennenden Zwischenräume verlaufen die Brechungsstege innerhalb der Transversalfläche zwangsweise zumindest näherungsweise parallel. Vorzugsweise sind die Brechungsstege hierbei aus Gold, Nickel oder Silizium gebildet. Die Zwischenräume sind wahlweise durch (luft- oder flüssigkeitsgefüllte) Lücken oder durch Zwischenstege aus einem optisch vergleichsweise dichten Material (also einem Feststoff mit für Röntgenstrahlen vergleichsweise großem Realteil des Brechungsindex), z.B. aus Photolack, gebildet. Dabei ist zu berücksichtigen, dass für Röntgenstrahlen der Realteil des Brechungsindex in allen Materialien kleiner als Eins ist, so dass für Röntgenstrahlen - anders als für sichtbares Licht - Feststoffe im Vergleich zu Vakuum oder Luft stets das optisch dünnere Medium darstellen.

Die von den Brechungsstegen innerhalb der Transversalfläche jeweils eingenommenen Flächenbereiche sind dabei typischerweise nicht deckungsgleich mit den Brechungsstreifen, die nur durch die strahlungsbrechenden Eigenschaften des Brechungsgitters definiert sind.

Gemäß einer in der WO 2013/160 153 A1 nicht beschriebenen Ausführungsalternative sind die Brechungsstege des Linsengitters $G_L$ zum Beispiel derart ausgebildet, dass sie innerhalb der Transversalfläche zumindest abschnittsweise diagonal (also schrägwinklig, d.h. unter einem 0° übersteigenden und 90° unterschreitenden Winkel zu der y-Achse) verlaufen. Der zumindest abschnittsweise diagonale Verlauf der Brechungsstege in der Transversalfläche ist hierbei dadurch charakterisiert, dass für mindestens einen Brechungssteg die diesen Brechungssteg in x-Richtung begrenzenden Seitenflächen sich innerhalb der Transversalfläche über mindestens zwei Brechungsstreifen erstrecken. Da die Brechungsstege, bedingt durch die Zwischenräume, zumindest näherungsweise parallel verlaufen müssen, erstreckt sich diese Eigenschaft zwangsweise auf alle Brechungsstege (mit Ausnahme von etwaigen Brechungsstegen an den Rändern der Transversalfläche, deren Seitenfläche sich aufgrund der Randlage nur über einen Brechungsstreifen erstrecken kann). Vorzugsweise erstrecken sich - von etwaigen Randeffekten abgesehen - die Seitenflächen aller Brechungsstege regelmäßig über eine Vielzahl von Brechungsstreifen, insbesondere über alle Brechungsstreifen.

In anderer, aber inhaltlich völlig äquivalenter Formulierung ist der "diagonale Verlauf" der Brechungsstege in der Transversalfläche dadurch charakterisiert, dass sich mindestens ein Brechungssteg über mindestens vier Brechungsstreifen erstreckt. Auch diese Eigenschaft gilt aufgrund des zumindest näherungsweisen Parallelverlaufs der Brechungsstreifen - von etwaigen Randeffekten abgesehen - zwangsweise für alle Brechungsstege.

Durch die vorstehend beschriebenen Eigenschaften unterscheiden sich das "diagonale Layout" der Brechungsstege der Ausführungsalternative des Linsengitters $G_L$ qualitativ von dem "streifenförmigen Layout" der Brechungsstege der in WO 2013/160 153 A1 angegebenen Linsengitter $G_L$. Dort verlaufen die Brechungsstege in Längsrichtung der Brechungsstreifen, d.h. in y-Richtung. Dabei bleiben die Seitenflächen eines jeden Brechungsstegs stets jeweils innerhalb des einem einzigen Brechungsstreifen zugeordneten Raums, wodurch sich die streifenförmigen Brechungsstege nur über zwei oder maximal drei Brechungsstreifen erstrecken können.

Wie bereits in WO 2013/160 153 A1 beschrieben ist, kann das Linsengitter $G_L$ erfindungsgemäß anstelle des Analysegitters $G_1$ des herkömmlichen Talbot-Lau-Interferometers eingesetzt werden. Das Linsengitter $G_L$ wird hierbei in seinem Fokusabstand zu dem Röntgendetektor angeordnet, so dass die Röntgenstrahlung durch das Linsengitter $G_L$ direkt auf die einzelnen Pixel oder Pixelspalten des Röntgendetektors fokussiert wird. Alternativ hierzu kann das Linsengitter $G_L$, wie ebenfalls bereits in WO 2013/160 153 A1 beschrieben ist, dem Phasengitter und dem Analysegitter zwischengeordnet werden. In diesem Fall wird die Röntgenstrahlung durch das Brechungsgitter auf die Schlitze und Stege des Analysegitters fokussiert.

Durch die Eigenschaft des Linsengitters $G_L$, jeweils mehrere benachbarte, einander entsprechende Strukturen (z.B. Intensitätsmaxima oder Intensitätsminima) des Interferenzmusters auf einen gemeinsamen Fokus zu brechen, wirkt das Linsengitter $G_L$ als periodische Anordnung von Sammellinsen, durch die das Interferenzmuster vergröbert wird. Dies ermöglicht den Einsatz eines entsprechend gröberen und in den Gitterstegen besser absorbierenden Analysegitters, wodurch die Sichtbarkeit (Visibilität) verbessert wird. Dies ermöglicht wiederum - insbesondere bei hochenergetischer (kurzwelliger) Röntgenstrahlung - eine Reduzierung des Bildrauschens und der Röntgendosis. Wird in alternativer Ausführung das Analysegitter weggelassen, so entfällt die hierdurch verursachte Absorption, wodurch - insbesondere bei niederenergetischer (langwelliger) Röntgenstrahlung - ebenfalls eine Reduzierung des Bildrauschens und der Röntgendosis erreicht wird.

Da ein Linsengitter $G_L$ regelmäßig mit kleinerer Bauhöhe gefertigt werden kann als ein Analysegitter, kann die Streifenbreite des Linsengitters $G_L$ im Vergleich zu der typischen Streifenbreite eines Analysegitters kleiner gewählt werden. Hierdurch kann zwischen dem Phasengitter und dem Linsengitter $G_L$ ein größerer (d.h. einem höheren Vielfachen des Talbot-Abstands entsprechender) Abstand eingestellt werden als zwischen dem Phasengitter und dem Analysegitter eines gewöhnlichen Talbot-Lau-Interferometers. Hierdurch ergibt sich eine höhere (Winkel-)Empfindlichkeit, wodurch der Nachteil einer etwas geringeren Sichtbarkeit überwogen wird. Die ermöglicht eine weitere Verbesserung des Bildrauschens und/oder eine weitere Verringerung der Röntgendosis.

Wie bereits in WO 2013/160 153 A1 beschrieben ist, ist das Linsengitter $G_L$ vorzugsweise in einem photolithographischen Herstellungsverfahren, insbesondere am sogenannten LIGA(Lithographie-Galvanik-Abformung-)-Verfahren oder mittels reaktivem Ionen-Ätzen hergestellt.

Ein einschränkender Faktor für die Herstellung der Linsengitter $G_L$ ist dabei das durch Herstellungsverfahren begrenzte Aspektverhältnis, das bei gegebener Gitterhöhe in z-Richtung durch die herstellbaren Minimalabstände zwischen den Seitenwänden der Brechungsstege bestimmt ist, nämlich - je nach dem konkreten Herstellungsverfahren - durch die minimale Stärke der Brechungsstege und/oder die minimale Stärke der Zwischenräume.

Erkanntermaßen können durch das diagonale Layout der Brechungsstege bei gegebener Gitterhöhe und gegebenen Brechungseigenschaften der Brechungsstreifen besonders große Minimalabstände zwischen den Seitenwänden der Brechungsstege - sowohl innerhalb der Brechungsstege als auch zwischen benachbarten Brechungsstegen - eingehalten werden. Dies ermöglicht wiederum die Fertigung von Linsengittern $G_L$ mit besonders großer Gitterhöhe in z-Richtung oder besonders geringer Breite der Brechungsstreifen. Solche Linsengitter $G_L$ ermöglichen die Realisierung von Phasenkontrast-Röntgenbildgebungsvorrichtungen mit besonders geringer Einbaulänge und besonders hoher Empfindlichkeit.

In bevorzugter Ausführung sind die Brechungsstege jeweils nach Art von in y-Richtung geneigten schiefen Prismen geformt, deren Grundfläche und Deckfläche jeweils in den zur Transversalfläche parallelen Stirnflächen des Linsengitters $G_L$ liegen. In dieser Ausführung wird das Linsengitter $G_L$ insbesondere, wie an sich bereits in WO 2013/160 153 A1 beschrieben ist, durch ein photolithographisches Verfahren, insbesondere LIGA, unter Schrägbelichtung der Photolackschicht durch Röntgenstrahlung hergestellt. Die Grundfläche und die gegenüberliegende Deckfläche des Prismas haben hierbei in der Regel jeweils eine komplexe, polygonale Form. An den Seitenrändern des Linsengitters $G_L$ können die Brechungsstege - abweichend von einer reinen Prismenform - zur Bildung von in z-Richtung ausgerichteten Randflächen abgeschnitten sein. Durch die Ausführung der Brechungsstege als zur Transversalfläche schiefe Prismen wird eine vergleichsweise starke Brechung erzielt, ohne andere optische Eigenschaften, insbesondere die Absorption und die Sichtbarkeit (Visibility) wesentlich zu verschlechtern.

Die Brechungsstege sind insbesondere derart angeordnet, dass in jedem Brechungsstreifen eine sich in y-Richtung mit einer y-Periodenlänge wiederholende Materialstruktur ergibt. Die Brechungsstege sind also derart gestaltet, dass sie in jedem Brechungsstreifen stets parallelverschobene, kongruente und gleichmäßig beabstandete Flächenabschnitte einnehmen. Die Brechungsstege sind dabei derart in y-Richtung geneigt, dass die zu der Grundfläche entgegengesetzte Deckfläche eines jeden Brechungsstegs gegenüber der Grundfläche um eine ganze Anzahl von Periodenlängen, insbesondere um genau eine Periodenlänge versetzt ist. Die beiden in z-Richtung gegenüberliegenden Stirnflächen des Linsengitters $G_L$ weisen somit ein identisches Layout, also eine identische aus Brechungsstegen und Zwischenräumen gebildete Materialstruktur auf.

Vorzugweise sind die Seitenflächen der Brechungsstege jeweils alternierend aus aktiven Teilflächen mit vergleichsweise starker Brechungswirkung in x-Richtung und passiven Teilflächen mit geringer oder verschwindender Brechungswirkung in x-Richtung zusammengesetzt. Die aktiven Teilflächen können hierbei als brechende Flächen von (Teil-)Prismen betrachtet werden, die mit ihren nicht-brechenden Rückenflächen zu einem den jeweiligen Brechungssteg bildenden Multi-Prisma zusammengesetzt sind.

Die aktiven und passiven Teilflächen sind hierbei vorzugsweise jeweils durch ebene (ungekrümmte) Flächenabschnitte gebildet. Die Brechungswirkung einer jeden Teilfläche wird dabei durch den zugehörigen Gradienten bestimmt. Als Gradient wird hierbei die Steigung $g = \Delta z / \Delta x$ bezeichnet, den diese Teilfläche in einem Schnitt entlang einer xz-Ebene (also einer durch die x-Achse und die z-Achse aufgespannten Ebene) aufweist. Je größer der Gradient g, je steiler also die jeweilige Teilfläche gegen die Transversalfläche angestellt ist, desto stärker wird das einfallende Röntgenlicht in x-Richtung gebrochen.

Jede aktive oder passive Teilfläche erstreckt sich innerhalb der Transversalfläche in x-Richtung dabei vorzugsweise über eine ganze Anzahl von Brechungsstreifen. Der Übergang zwischen aktiven und passiven Teilflächen einer Seitenfläche fällt somit vorzugsweise jeweils mit dem Übergang zwischen zwei Brechungsstreifen zusammen. Aktive und passive Teilflächen sind dabei vorzugsweise an den beiden Seitenflächen eines Brechungsstegs versetzt zueinander angeordnet. In einem Brechungsstreifen, in dem eine erste Seitenfläche eines jeden Brechungsstegs eine aktive Teilfläche aufweist, hat die andere Seitenfläche desselben Brechungsstegs somit eine passive Teilfläche und umgekehrt. Eine Ausnahme von dieser Regel bilden hierbei Brechungsstreifen ohne Brechungswirkung in x-Richtung (neutrale Brechungsstreifen), in denen beide Seitenflächen eines Brechungsstegs jeweils passive Teilflächen aufweisen.

Die passiven Teilflächen können exakt in der Transversalfläche parallel zur x-Achse ausgerichtet sein (g = 0). Vorzugsweise weisen auch die passiven Teilflächen aber einen kleinen Gradienten (Offset-Steigung) auf. Diese Offset-Steigung ist insbesondere derart bemessen, dass die dadurch verursachte Steigung $\Delta y$ der

passiven Teilflächen über einem Brechungsstreifen - in y-Richtung gemessen - etwa zwischen 20% und 50%, vorzugweise etwa 25% der in x-Richtung gemessenen Streifenbreite $s_L$ entspricht ($0,2 \leq \Delta y/s_L \leq 0,5$).

Durch die Offset-Steigung der passiven Teilflächen können bei gegebener Gitterhöhe und gegebenen Brechungseigenschaften der Brechungsstreifen die Minimal-Abstände zwischen den Seitenwänden der Brechungsstege - sowohl innerhalb der Brechungsstege als auch zwischen benachbarten Brechungsstegen - vorteilhafterweise weiter vergrößert werden.

Die Offset-Steigung der passiven Teilflächen bewirkt des Weiteren eine Abflachung der zwischen den aktiven und passiven Teilflächen gebildeten Winkel, was die technische Herstellbarkeit des Linsengitter $G_L$ begünstigt.

Da die Offset-Steigung jeweils die obere und die untere Materialkante einer Spalte gleichermaßen betrifft, ändert es die dadurch codierte Phasenverschiebung nicht. Aus einem Rechteck ("Gradient 0") wird durch die Offset-Steigerung ein Parallelogramm. Das Parallelogramm wirkt genauso auf die Phase des hindurchtretenden Lichts wie ein Rechteck. Die Offset-Steigung ist zweckmäßigerweise zwischen Oberkante und darunterliegender Unterkante des Materials gleich gewählt. Unterschiedliche Spalten dürfen auch unterschiedliche zusätzliche Gradienten erhalten für Kanten, die eine x-Komponente besitzen (rein vertikale Sprünge in y-Richtung werden nicht beeinflusst). Auch kann eine Spalte im linken und im rechten Teil (in x-Richtung) eine unterschiedliche Offset-Steigung besitzen, wenn dazwischen ein Knick erfolgt.

In einer zweckmäßigen Ausführungsform des Linsengitters $G_L$ verläuft jeder Brechungssteg innerhalb der Transversalfläche in alternierenden Abschnitten diagonal in positiver y-Richtung und in negativer y-Richtung. Die Brechungsstege weisen also Knickstellen auf. Bevorzugt sind die Brechungsstege in regelmäßigen Abständen entlang der x-Achse alternierend gegensätzlich geknickt, so dass der jeweilige Brechungssteg innerhalb der Transversalfläche mäandrierend in Richtung der x-Achse verläuft. Durch das ein- oder mehrfach geknickte Layout für die Brechungsstege werden bei der Herstellung des Linsengitters $G_L$ im LIGA-Verfahren die zunächst durch Belichtung mit Röntgenstrahlung und anschließende Entwicklung herausgearbeiteten Zwischenstege aus Photolack mechanisch stabilisiert. Die Knickstellen sind vorzugsweise jeweils im Bereich von neutralen oder schwach brechenden Brechungsstreifen vorgesehen. Der Verlauf eines jeden Brechungsstegs innerhalb der Transversalfläche ändert somit jeweils an Brechungsstreifen mit geringer oder verschwindender Brechungswirkung die Richtung.

Zweckmäßigerweise sind stets jeweils eine einheitlich vorgegebene Anzahl von Brechungsstreifen auf einen gemeinsamen Fokus ausgerichtet. Die auf einen gemeinsamen Fokus ausgerichteten Brechungsstreifen werden hierbei zusammenfassend als Fokussierungsgruppe bezeichnet. Die Fokussierungsgruppen umfassen hierbei vorzugsweise jeweils eine ungeradzahlige Anzahl von Brechungsstreifen, z.B. 3, 5, 7 oder 9 Brechungsstreifen. Eine solche Fokussierungsgruppe umfasst einen neutralen Brechungsstreifen mit verschwindender Brechungswirkung, um den herum symmetrisch die weiteren Brechungsstreifen der Fokussierungsgruppe angeordnet sind, wobei deren Brechungswirkung in x-Richtung mit zunehmendem Abstand zu dem neutralen Brechungsstreifen ansteigt. Die Brechungsstreifen benachbarter Fokussierungsgruppen sind hierbei ineinander verschachtelt.

In bevorzugten Ausführungsformen des Linsengitter $G_L$ sind ferner ein oder mehrere der vorstehend beschriebenen Ausgestaltungsmerkmale vorgesehen, um die Minimal-Abstände innerhalb der Brechungsstege und zwischen benachbarten Brechungsstegen weiter zu vergrößern und/oder um die optischen Eigenschaften des Linsengitter $G_L$ zu optimieren:

- F1 - "Gradientenkompression": Dieses Ausgestaltungsmerkmal wird insbesondere auf die stärksten Teilprismen (also die aktiven Teilflächen der Brechungsstege mit den jeweils größten Gradienten) angewandt, wenn bei gegebener Gitterhöhe h und gegebener Streifenbreite $s_L$ der Brechungstreifen durch eine aktive Teilfläche, die innerhalb der xz-Ebene linear über die gesamte Streifenbreite $s_L$ und Gitterhöhe h verläuft ($g = h/s_L$), eine hinreichend starke Brechung nicht erzielt werden kann. Die Gradientenkompression wird realisiert, indem die aktive Teilfläche nicht über die volle Streifenbreite $s_L$ geführt ist, sondern lediglich über einen Anteil $1/c$ (mit $c > 1$) dieser Streifenbreite $s_L$, während sich die aktive Teilfläche in z-Richtung vorzugsweise über die gesamte Gitterhöhe h erstreckt. Der Gradient g erhöht sich in diesem Fall auf $g = c \cdot h/s_L$. An der maximalen Visibilität ändert die Gradientenkompression zumindest dann wenig, wenn die Intensität in dem verbleibenden Rand des Brechungsstreifens relativ gering ist. Wenn beispielsweise die Streifen hoher Intensität des durch das Phasengitter $G_1$ gebildeten Interferenzmusters in dem Brechungsgitter $G_L$ genau auf einen Streifen fallen, so fließt die Hauptintensität in der Streifenmitte (=Spaltmitte) und der Rand bleibt vergleichsweise dunkel. Durch den durch Gradientenkompression erhöhten Gradient wird ermöglicht, den Abstand zwischen dem Patienten und dem Röntgendetektor vergleichsweise klein zu halten, was auch die Empfindlichkeit verbessert. Der kleinere Abstand führt dazu, dass jeder Brechungsstreifen auf einen schmäleren Bereich abgebildet wird, dies verbessert die Sichtbarkeit für geringe Kompression (z.B. bei $c^2 < 2$). Bei größerer Kompression (z.B. $c^2 > 2$) überwiegt dagegen die Reduktion der Sichtbarkeit durch die Randverluste in den am stärksten brechenden Teilprismen. In zweckmäßiger Dimensionierung ist c im Bereich 4/3

$\leq c \leq 3/2$ wie z.B. $c = 2^{1/2}$ gewählt. Die Gradientenkompression wird vorzugsweise symmetrisch bezüglich des zugehörigen Brechungsstreifens durchgeführt. Die in ihrer Breite (in x-Richtung) reduzierte aktive Teilfläche wird also bezüglich des zugehörigen Brechungsstreifens zentriert.

Variante: Eine aus der Anmeldung US 2012/0041679 A1 beschriebene Methode zielt darauf ab, alle Gitter bei einem auf Phasenkontrastbildgebung beruhenden Computertomographen so auszurichten, dass in Abwesenheit des Patienten im Strahl die hellen Streifen des von dem Phasengitter $G_1$ erzeugten Interferenzmusters genau auf die Streifengrenzen des Analysegitters ausgerichtet sind. Bei einer vollen Umdrehung des Computertomographen (in Anwesenheit des Patienten) wird dann durch den Patient der Streifen einmal nach z.B. rechts und (nach 180° Gantry-Drehung) einmal in die Gegenrichtung (hier z.B. nach links) verschoben. Dies kann ohne jede Gitterverschiebung abgetastet und in Bilder umgerechnet werden. In Übertragung dieser Idee auf die erfindungsgemäße Phasenkontrast-Röntgenbildgebungsvorrichtung wird die Gradientenkompression zweckmäßigerweise asymmetrisch durchgeführt. In diesem Fall wird zweckmäßigerweise vorher festgelegt, welche Spaltgrenzen des Linsengitters $G_L$ die hohe Intensität erhalten sollen. Der komprimierte Gradient wird von der Streifenmitte an diese Spaltgrenze verschoben. Die asymmetrische Kompression hat also den gradientenfreien Teil der Breite $s_L(1-1/c)$ zusammenhängend auf der zunächst unbeleuchteten Seite des Brechungsstreifens und den Gradienten bis zum Rand der beleuchteten Spaltengrenze.

- F2 - Gradienten-Variation: Bei den in WO 2013/160153 A1 beschriebenen Linsengittern $G_L$ sind die Teilprismen bzw. Teilflächen stets derart ausgerichtet, dass alle Teilprismen bzw. Teilflächen eine Design-Wellenlänge $\lambda_D$ bzw. Design-Photonenenergie möglichst exakt auf einen gemeinsamen Fokus fokussieren, in dem die Mitte des zugehörigen Elements des Analysegitters $G_2$ (Spalt bzw. Steg) bzw. Detektor-Pixels platziert wird. Dies beschränkt allerdings das Layout stärker als notwendig:

   Die stärksten Teilprismen verteilen aufgrund von Dispersion die Licht-Intensität über einen weiten Bereich (oft über mehr als eine Pixelbreite bzw. $S_2$-Spaltbreite). Ihre Gradienten sind daher nicht variierbar ohne weitere Sichtbarkeitsverluste in den zugehörigen Brechungsstreifen. Je schwächer jedoch die Teilprismen werden (je näher die Teilprismen entlang der x-Achse an dem zugehörigen Fokus liegen), desto näher zueinander werden die einzelnen Wellenlängen abgebildet, desto schmäler wird also insgesamt der beleuchtete Bereich. Dies bietet die Freiheit, den Gradienten dieser Prismen leicht zu erhöhen oder zu verringern, ohne die Sichtbarkeit signifikant zu verschlechtern. Die schwächsten Teilprismen befinden sich regelmäßig (entlang der x-Achse) zwischen den stärksten Teilprismen und den zweitstärksten Teilprismen der benachbarten Fokussierungsgruppe. Hier lassen sich durch leichte Verstärkung dieser schwächsten Teilprismen die Minimal-Abstände innerhalb der Brechungsstege und zwischen den Brechungsstege vergrößern, indem die zugehörigen Teilflächen in z-Richtung gestaucht werden. Oft können auch Gradienten nunmehr gleich starker Nachbar-Teilprismen reduziert werden, was die Absorption durch das Linsengitter $G_L$ reduziert.

- F3 - Variation der Grundhöhe: Die Grundhöhe (d.h., der über die volle Streifenbreite im Layout vorhandene rechteckige oder parallelogrammförmige Teil des Materials) kann verändert werden, um das Material-Aspektverhältnis zu verbessern. Das Aspektverhältnis wird hierbei verringert, indem die Grundhöhe durch Hinzufügen von Material im Layout über die volle Streifenbreite erhöht wird. Alternativ hierzu kann das Freiraum-Aspektverhältnis verbessert werden, indem die Grundhöhe reduziert wird - dies kann bei gegebenem und übererfülltem Aspektverhältnis auch zur Reduzierung des Material-Anteils und damit der Absorption genutzt werden. Diese Änderung betrifft vorwiegend schwache Teilprismen (mit kleinem Gradienten) und mittelstarke Teilprismen.

   Bei schwachen Teilprismen empfiehlt sich, zur materialsparenden Optimierung des Aspektverhältnisses, anstelle einer Erhöhung der Grundhöhe eine Gradientenerhöhung (siehe F2) einzusetzen, solange Sichtbarkeit nicht signifikant reduziert ist.

- F4 - Zentrale Spalte: Bei den in WO 2013/160153 beschriebenen Linsengittern $G_L$ ist der zentrale (und optisch neutrale) Brechungsstreifen einer jeden Fokussierungsgruppe stets komplett mit Material gefüllt. Dies bedingt eine vergleichsweise starke Absorption in den zentralen Brechungsstreifen, wo eigentlich aus optischen Gründen gar nichts erforderlich wäre (da in den zentralen Brechungsstreifen keinerlei Gradient realisiert werden muss).

Hier ist es denkbar, den Aufbau an der zentralen Spalte komplett zu spiegeln (d.h., Aufbau achsensymmetrisch zur y-Achse in der Mitte der zentralen Spalte) und dafür rechteckige Materiallücken über die volle Breite der zentralen Brechungsstreifen (oder etwas mehr oder etwas weniger) einzubauen. Diese Lücken sind von der Brechung her neutral, reduzieren aber die Absorption. Sie sind so zu bemessen, dass minimale Materialbreiten nicht unterschritten werden. Vorzugsweise entsprechen sie einer Phasenverschiebung des hindurchtretenden Lichtes entlang der z-Achse um ein ganzzahliges Vielfaches an vollen Wellenlängen (analog F6 bzw. F7, für eine Wellenlänge des Spektrums nahe der Design-Wellenlänge).

- F5 "Gradienten-Verschmälerung": Diese Maßnahme ist sehr ähnlich zu der unter Punkt F1 beschriebenen Gradientenkompression, wird aber nicht auf die stärksten Teilprismen, sondern vorwiegend auf die schwächsten Teilprismen angewandt. Wird deren Gradient im streifenförmigen Layout am äußeren und/oder inneren Material-Rand in Richtung der Streifenmitte des Brechungsstreifens mit Streifenbreite $s_L$ zurechtgeschnitten, so können damit die Mindestabstände erhöht werden.

Wird der äußere Rand zurechtgeschnitten (das sei der Rand, in dem sich in y-Richtung wenig oder kein Material befindet), so wird der mäanderförmige Materialstreifen aus zwei benachbarten Spalten insgesamt schmäler. Der Freiraum-Mindestabstand kann so zunehmen. Wird der innere Rand zurechtgeschnitten (das sei der Rand, in dem sich in y-Richtung viel Material befindet), so wird letztlich die Spaltengrenze zwischen den benachbarten Brechungsstreifen des mäanderförmigen Materialstreifens in Richtung des schwächeren Teilprismas verschoben. Der Material-Mindestabstand kann auf diese Weise vergrößert werden. Dabei sollte (um die maximale Sichtbarkeit wenig zu beeinflussen) maximal 10-15% der Streifenbreite beeinflusst werden.

- F6 - Dispersionskorrektur: Durch die unter Punkt F1 erläuterte Gradientenkompression wird eine Erhöhung des geometrischen Gradienten der stärksten Teilprismen erreicht. Allerdings nimmt die Licht-Ablenkung der Brechungsstreifen nicht in demselben Maße zu. Dieser Effekt lässt sich zur Dispersionskorrektur bei den stärksten Teilprismen einsetzen. Da der Brechungsindex $\delta \propto \lambda^2 \propto 1/E^2$ und der Ablenkwinkel $\gamma \approx \delta \times g$ ist, folgt, dass $\gamma \propto \lambda^2 \propto 1/E^2$ ist. Gegeben sei ein starker Gradient, der die Materialhöhe (und damit die Phase aufgrund der höheren Phasengeschwindigkeit durch das Material) in Richtung positiver x-Achse steigert (und das Licht damit in negativer x-Richtung ablenkt). Der x-Abstand $\Delta x$ sei nun passend zum Gradient g so gewählt, dass $\Delta\Phi = 2\pi (\delta/\lambda)g \Delta x = 2\pi$ gilt, dass sich also entlang von $\Delta x$ die Phase durch den Gradient genau um $\Delta\Phi = 2\pi$ ändert bzw. die Wellenfront um $\Delta z = +\lambda_D$ in Ausbreitungsrichtung verschoben wird (für eine vorzugsweise nahe der Mitte des Auslegungs-Spektrums liegende einer Design-Wellenlänge $+\lambda_D$). Wenn hier in der positiven x-Richtung der steigender Materialhöhe nun die Materialhöhe sprunghaft um $\Delta h = g \cdot \Delta x$ reduziert wird, so dass ein Phasensprung von $\Delta\Phi = -2\pi$ bzw. $\Delta z = -\lambda_D$ entsteht, so ist dies für die Design-Wellenlänge $\lambda_D$ ohne jede Auswirkung (da nur die Phase modulo $2\pi$ relevant ist). Für eine um einen Faktor f geänderte Wellenlänge $X = f \cdot \lambda_D$ gilt jedoch $\delta(\lambda)/\lambda \propto f \cdot \lambda_D$, so dass wegen $\Delta\Phi = 2\pi (\delta/\lambda)\Delta h$ lokal an der Sprungstelle eine Phasen-Abweichung von $2\pi \cdot (1-f)$ verbleibt. Falls man Abweichungen von $\pm\pi/4$ toleriert, so muss also $3/4 \leq f \leq 5/4$ gelten. Das Spektrum hat also eine Bandbreite vom Faktor 5/3=167% (was gut zur bisherigen Bandbreite des Linsengitters $G_L$ vom Faktor $3^{1/2}$=173% passt). Wird alle $\Delta x$ ein dem Gradient entgegengesetzter Phasensprung erzeugt (wodurch die Materialhöhe eine Sägezahnkurve ohne jede mittlere Steigung beschreibt), so kompensieren sich jedoch die Phasen-Abweichung von $2\pi$ durch den Gradient mit der durch den Phasensprung, so dass sich die Phase (bis auf die lokale Abweichung von $2\pi \cdot (1-f)$) im Mittel durch die Gerade $\Delta\Phi = 2\pi \cdot x/\Delta x$ annähern lässt, und zwar unabhängig von f bzw. $\lambda$. Für die Röntgenstrahlung sieht es also wie eine fehlerbehaftete kontinuierliche Steigung bei $\delta \propto \lambda^1 \propto 1/E^1$ aus. Es resultieren Ablenkungswinkel linear zu f: $\gamma \propto \lambda^1$ oc $1/E^1$ oc f. Der Farbfehler kann so also um eine Größenordnung reduziert werden. Wird in kürzeren Abständen als $\Delta x$ ein Phasensprung eingefügt, so wird ggf. überkompensiert, was in geringem Umfang sinnvoll sein kann. Es empfiehlt sich der Einsatz von Phasensprüngen erst zur Korrektur von Farbfehlern, wenn $(\delta/\lambda)g \cdot s_L$ größer oder zumindest nicht wesentlich kleiner als 1 ist.

Zahlenbeispiele: Für E=62 keV-Photonen entspricht ein voller Phasensprung einer Gold-Höhe von h=25 $\mu$m (Transmission 85%) oder einer Nickel-Höhe von 43 $\mu$m (Transmission 95%). Bei E=29 keV dagegen ist es eine Nickel-Höhe von 20 $\mu$m (Transmission 82%) oder eine Silizium-Höhe von 75$\mu$m (Transmission 98%). Bei E=19 keV Photonen entspricht der Sprung um eine Phase einer Silizium-Höhe von 50 $\mu$m (Transmission 95%).

Ein Problem dieser Strategie ist es, dass an der Sprungstelle genug Material verbleiben muss, um die Mindestbreiten des Layouts zu erfüllen. Deshalb spart der negative Phasensprung einerseits Material, es muss andererseits aber auch Material hinzugefügt werden, um an den Sprungstellen noch genug Material zu haben. Letzten Endes kann das bedeuten, dass man Material hinzufügen muss.

- F7 - Zusätzliche Phasensprünge: Im Gegensatz zu der unter Punkt F6 diskutierten Dispersionskorrektur wird hier die Dispersion erhöht (und ist bei kleinen Gradienten dann näherungsweise unabhängig von einer Variation des Gradienten). Zweckmäßigerweise wird die die Dispersion der schwächsten Teilprismen durch einen oder mehrere zusätzliche 2n-Sprünge (Sprünge um jeweils eine Wellenlänge) erhöht. Dadurch nimmt der Materialanteil im Layout zunächst zu, und der Material-Mindestabstand kann dadurch - erwünschtermaßen - auch zunehmen. Diese Maßnahme erlaubt allerdings, die unter Punkt F3 diskutierte Grundhöhe zu reduzieren und so den Freiraum-Mindestabstand zu reduzieren. Letztlich nähert sich die Material/Freiraum-Grenze im Streifen der schwächsten Teilprismen damit der Material/Freiraum-Grenze der gegenüberliegenden stärksten Teilprismen in grober Näherung an. Durch die Wahl der x-Positionen der λ-Phasensprünge innerhalb des Streifens kann man beeinflussen, ob der Freiraum-Abstand (in Richtung der gegenüberliegenden stärksten Teilprismen) stärker oder weniger stark als der Materialabstand (innerhalb des mäanderförmigen Materialstreifens mit der benachbarten Prismenspalte) beeinflusst werden soll. Bei größeren Gitterhöhen kann es auch sinnvoll sein, mehrere komplette Phasensprünge relativ gleichmäßig über die Spaltenbreite zu verteilen (z.B. drei Phasensprünge an den Positionen 12,5%, 50%, 87,5% der Teilprismenbreite oder an den x-Positionen 25%, 50%, 75% von $s_L$). Dies gilt entsprechend auch für die unter Punkt F6 diskutierte Dispersionskorrektur.

Die unter den Punkten F1 bis F6 diskutierten Ausgestaltungsmerkmale der Brechungsstege und der darin enthaltenen aktiven Teilflächen (Teilprismen) können vorteilhaft sowohl bei diagonalen Layouts der Brechungsstege als auch bei streifenförmigen Layouts gemäß WO 2013/160153 A1 eingesetzt werden, um die Minimal-Abstände innerhalb der Brechungsstege und zwischen benachbarten Brechungsstegen weiter zu vergrößern und/oder um die optischen Eigenschaften des Linsengitters $G_L$ zu optimieren. Jedes der unter den Punkten F1 bis F6 diskutierten Merkmale wird daher auch für sich gesehen (unabhängig von dem großräumigen Verlauf der Brechungsstege in der Transversalfläche) als eigenständige Erfindung betrachtet.

Wie zuvor beschrieben sind bei einem Linsengitters $G_L$ stets jeweils eine einheitlich vorgegebene Anzahl von Brechungsstreifen auf einen gemeinsamen Fokus ausgerichtet, wobei die auf einen gemeinsamen Fokus ausgerichteten Brechungsstreifen zusammenfassend als Fokussierungsgruppe bezeichnet werden. Eine solche Fokussierungsgruppe umfasst einen neutralen Brechungsstreifen mit verschwindender Brechungswirkung, um den herum symmetrisch weitere Brechungsstreifen der Fokussierungsgruppe angeordnet sind, wobei deren Brechungswirkung in x-Richtung mit zunehmenden Abstand zu dem neutralen Brechungsstreifen ansteigt. Im Falle der Brechungsgitter $G_A$ und/oder $G_B$ hingegen brechen die aufeinanderfolgenden Brechungsstreifen abwechselnd nach links und nach rechts, also in Richtung der positiven oder der negativen x-Achse, und die Brechwirkung in Richtung der x-Achse aufeinanderfolgender Brechungsstreifen wechselt lediglich alternierend zwischen zwei benachbarten Brechungsstreifen. Dementsprechend sind bei einem Brechungsgitter $G_A$ und/oder $G_B$ nicht mehrere Brechungsstreifen auf einen gemeinsamen Fokus hin ausgerichtet und dementsprechend sind auch keine unterschiedlichen Brechstärken für verschiedene Brechungsstreifen realisiert. Man könnte quasi sagen, dass die Brechungsgitter $G_A$ und $G_B$ einen Grenzfall des Linsengitters $G_L$ darstellen, bei dem jede Fokussierungsgruppe durch einen einzigen Brechungsstreifen gegeben ist.

Wie bereits ausführlich dargelegt, dient der Phasenkontrast-Differenzverstärker zur Erhöhung der Empfindlichkeit der Phasenkontrast-Röntgenbildgebungsvorrichtung. Dagegen wird ein zuvor beschriebenes Linsengitter $G_L$ genutzt, um die Sichtbarkeit zu erhöhen, wie dies in der WO 2013/160 153 A1 beschrieben ist. Gemäß einer vorteilhaften Ausgestaltungsvariante der Phasenkontrast-Röntgenbildgebungsvorrichtung werden beide Prinzipien miteinander kombiniert und dementsprechend weist die Phasenkontrast-Röntgenbildgebungsvorrichtung in diesem Fall einen Phasenkontrast-Differenzverstärker auf und zudem ist zwischen dem Phasenkontrast-Differenzverstärker und dem Röntgendetektor ein zusätzliches Brechungsgitter angeordnet, welches nach Art eines zuvor beschriebenen Linsengitters $G_L$ ausgestaltet ist.

Ausführungsbeispiele der Erfindung werden nachfolgend anhand einer schematischen Zeichnung näher erläutert. Darin zeigen:

FIG 1      in einer schematischen Darstellung eine Phasenkontrast-Röntgenbildgebungsvorrichtung,

FIG 2      in einer schematischen Darstellung ein klassisches Talbot-Lau-Interferometer,

FIG 3      in einer schematischen Darstellung eine alternative Ausführung der PhasenkontrastRöntgenbildgebungsvorrichtung,

FIG 4      in einer schematischen Darstellung eine zweite alternative Ausführung der PhasenkontrastRöntgenbildgebungsvorrichtung,

FIG 5      in einer ausschnittsweisen Darstellung ein Phasenkontrast-Differenzverstärker,

FIG 6      in einer schematischen Darstellung eine alternative Ausführung des Phasenkontrast-Differenzverstärker,

FIG 7      in einer schematischen Darstellung eine zweite alternative Ausführung des PhasenkontrastDifferenzverstärker,

FIG 8      in einer schematischen Darstellung eine dritte alternative Ausführung des Phasenkontrast-Differenzverstärker,

FIG 9      in einer schematischen Darstellung eine vierte alternative Ausführung des Phasenkontrast-Differenzverstärker,

FIG 10   in einer schematischen Darstellung ein Layout für eine Herstellung eines PhasenkontrastDifferenzverstärkers,

FIG 11   in einer schematischen Darstellung ein alternatives Layout für eine Herstellung eines PhasenkontrastDifferenzverstärkers und

FIG 12   in einer schematischen Darstellung ein zweites alternatives Layout für eine Herstellung eines Phasenkontrast-Differenzverstärkers.

Einander entsprechende Teile sind in allen Figuren jeweils mit den gleichen Bezugszeichen versehen.

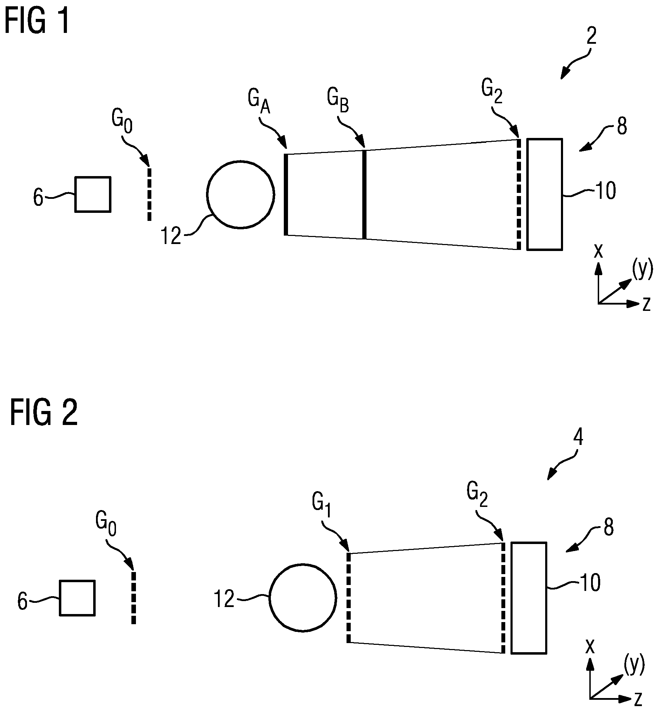

Eine nachfolgend exemplarisch beschriebene und in FIG 1 skizzierte Phasenkontrast-Röntgenbildgebungsvorrichtung 2 umfasst, genau wie ein in FIG 2 schematisch dargestelltes klassisches Talbot-Lau-Interferometer 4, eine Röntgenröhre 6, ein Kohärenzgitter $G_0$, ein Analysegitter $G_2$ sowie einen Röntgendetektor 8 mit einer regelmäßigen Anordnung von Detektorpixeln 10, die entlang einer z-Achse angeordnet sind. Zudem ist zwischen der als Röntgenstrahlungsquelle dienenden Röntgenröhre 6 und dem Röntgendetektor 8 eine Patientenliege 12 positioniert, die als Aufnahme für einen Patienten dient, welcher mittels der Phasenkontrast-Röntgenbildgebungsvorrichtung 2 und somit mittels einer Phasenkontrastbildgebung untersucht werden soll.

Wie aus FIG 1 hervorgeht, ist das Phasengitter $G_1$ des klassischen Talbot-Lau-Interferometers 4 in der Phasenkontrast-Röntgenbildgebungsvorrichtung 2 durch zwei Brechungsgitter $G_A$ und $G_B$ ersetzt, die zusammen einen Phasenkontrast-Differenzverstärker 14 ausbilden.

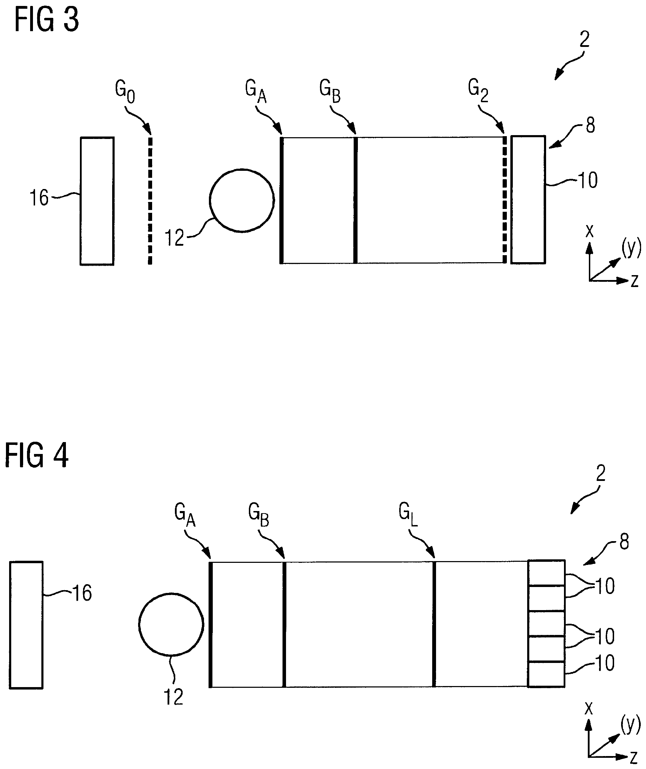

Der in FIG 1 skizzierte Aufbau der Phasenkontrast-Röntgenbildgebungsvorrichtung 2 lässt sich dabei für verschiedene Anwendungszwecke variieren und zwei alternative Aufbauten sind FIG 3 und FIG 4 angedeutet. In beiden Fällen dient eine Synchrotron-Strahlungsquelle 16 anstelle der Röntgenröhre 6 als Röntgenstrahlungsquelle, wobei im Falle der Ausführungsvariante gemäß FIG 4 eine Synchrotron-Strahlungsquelle 16 gegeben ist, die in sehr guter Näherung Röntgenstrahlung mit ebenen Wellenfronten generiert, so dass bei diesem Aufbau auf ein Kohärenzgitter $G_0$ verzichtet werden kann. Zudem ist bei diesem Aufbau das Analysegitter $G_2$ durch ein Linsengitter $G_L$ ersetzt, wie es aus der WO 2013/160 153 A1 bekannt ist.

Wird hingegen ein Kohärenzgitter $G_0$ mit der Periode $p_0$ genutzt, so darf bei diesem im Wesentlichen nur jeder 2N-te Spalt geöffnet sein, wobei N die Zahl der Streifen der Breite $s_B$ an der z-Position entlang der z-Achse von $G_B$ ist, so dass die an der z-Position von $G_A$ in Streifen der Breite $s_A$ eintretende elektromagnetische Strahlung verschoben wird bis es bei $G_B$ austritt. Gegenüber einem klassischen Talbot-Lau-Interferometers ist dabei die Periode $p_0$ um denselben Faktor 2N verkleinert. Der Grund hierfür ist, dass der Kontrast oder die Intensität um diesen Faktor schneller zu- und abnimmt (wenn $G_0$ verschoben wird oder in Richtung des eingehenden Strahls gedreht wird) verglichen mit dem klassischen Talbot-Lau-Interferometer. Wenn mehrere nebeneinander liegende Spalten geöffnet sind, so macht sich dies bezüglich der Empfindlichkeit des Talbot-Effekts zwischen $G_B$ und $G_2$ zunehmend störend bemerkbar. Diese Problematik ist in einem etwas anderen Kontext bereits in der WO 2013/160 153 A1 beschrieben.

Gemäß einer weiteren, nicht näher dargestellten Ausführungsvariante sind die zwei Aufbauten aus FIG 1 und FIG 2 in einer gemeinsamen Bildgebungsvorrichtung, also beispielsweise einem Computertomographen realisiert und werden dabei im Wechsel eingesetzt. Dementsprechend wird dann eine Messung mit dem Aufbau mit Phasenkontrast-Differenzverstärker 14 vorgenommen, um bei niedriger Sichtbarkeit aber hoher Empfindlichkeit kleine Phasenänderungen zu erfassen (bei größeren Phasenänderungen tritt Face Rapping auf und der Messfehler wird gegebenenfalls nicht bemerkt) und parallel oder leicht zeitversetzt hierzu wird eine zusätzliche Messung vorgenommen mit dem Aufbau ohne Phasenkontrast-Differenzverstärker 14, um bei reduzierter Empfindlichkeit aber höherer Sichtbarkeit größere Phasenänderungen korrekt zu messen (bei kleineren Phasenänderungen hat diese Messmethode wegen der geringeren Empfindlichkeit ein höheres Rauschen und ist deshalb weniger geeignet).

Hierzu sind die beiden Aufbauten beispielsweise um 90° versetzt zueinander z.B. in einem Computertomographen eingebaut (hier kann für jede Messung die gesamte Röntgendosis frei auf beide Messverfahren verteilt werden). Alternativ lassen sich die beiden Aufbauten auch miteinander verschmelzen, wobei zum Beispiel der Detektorbereich des Computertomographen in einer Richtung parallel zur Wirbelsäule eines Patienten aufgeteilt wird in ein Band für die eine Messung und in ein Band für die andere Messung (wenn der Tischvorschub des Patiententisches entsprechend reduziert wird und mehr Umdrehungen pro Vorschub zugelassen werden). Diese Variante eignet sich insbesondere, wenn ohne Phase-Stepping gearbeitet wird.

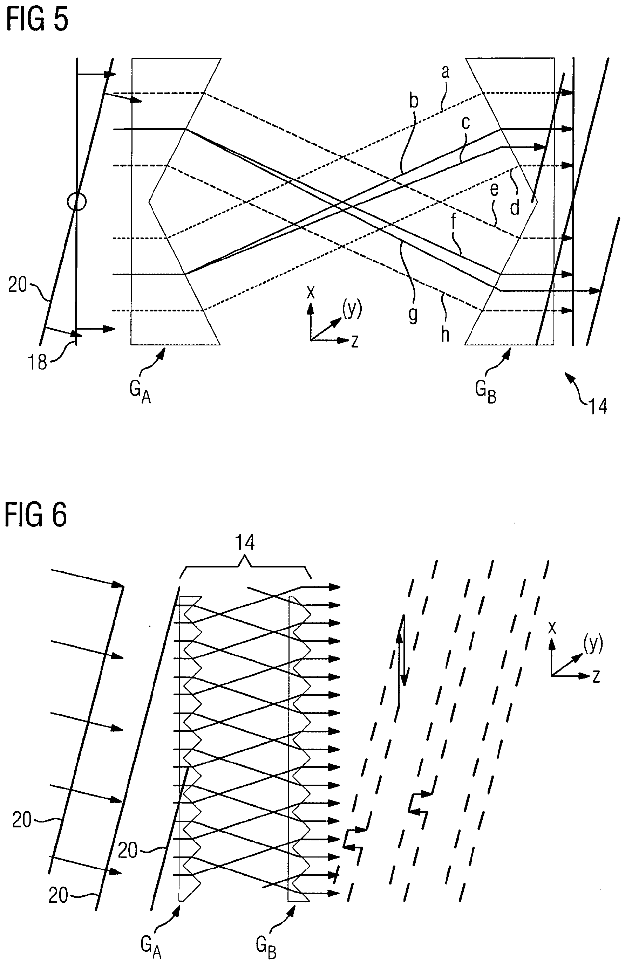

Der in der Phasenkontrast-Röntgenbildgebungsvorrichtung 2 eingesetzte Phasenkontrast-Differenzverstärker 14 ist aus zwei gleichartigen Brechungsgittern $G_A$ und $G_B$ aufgebaut und dient zur Verstärkung lokaler, durch ein Untersuchungsobjekt, also durch einem Patienten, hervorgerufener Phasenunterschiede und somit zur Erhöhung des Auflösevermögens der Phasenkontrast-Röntgenbildgebungsvorrichtung 2. Das grundlegende Funktionsprinzip lässt sich dabei anhand der Darstellung in FIG 5 aufzeigen.

Hier ist ein Art Basiseinheit des Phasenkontrast-Differenzverstärkers 14 gezeigt, auf welche von links einmal eine unbeeinflusste Wellenfront 18 auftrifft und einmal eine durch den Patienten beeinflusste Wellenfront 20, deren Richtung sich durch einen von Null abweichenden Phasengradienten beschreiben lässt.

Betrachtet man nun verschiedene Strahlengänge a bis h durch den Phasenkontrast-Differenzverstärker 14 so erkennt man, dass im Falle der unbeeinflussten Wellenfront 18 die Summe der beiden Wegstrecken durch das brechende Material der beiden Brechungsgitter $G_A$ und $G_B$ unabhängig von der x-Position ist, an der der Strahl auf das Brechungsgitter $G_A$ auftrifft, so dass hier eine einheitliche Phasenverschiebung erfolgt.

Dabei ist stets die Summe der beiden Teilstrecken durch das brechende Material bei $G_A$ einerseits und bei $G_B$ andererseits zu betrachten. Die Wegstrecke durch das brechende Material eines einzigen Brechungsgitters, also entweder $G_A$ oder $G_B$, hingegen variiert durchaus in Abhängigkeit der x-Position. Durch die Kombination der beiden Brechungsgitter $G_A$ und $G_B$ werden diese Unterschiede jedoch wieder ausgeglichen, d.h. wenn die Wegstrecke durch das brechende Material eines Strahlengangs beim Brechungsgitter $G_A$ geringer ausfällt, so ist die Wegstrecke durch das brechende Material beim Brechungsgitter $G_B$ entsprechend größer und umgekehrt, was der Vergleich der Strahlengänge a oder h und d oder e zeigt.

Trifft die Wellenfront hingegen unter einem bestimmten Winkel auf das Brechungsgitter $G_A$ auf, wie im Falle der beeinflussten Wellenfront 20, so ändert sich die Wegstrecke durch das brechende Material in Abhängigkeit der x-Position. So ist im Falle des Strahlengangs c die gesamte Materialhöhe beim Durchgang durch das Brechungsgitter $G_A$ und durch das Gitter $G_B$ vermindert und beim Strahlengang g vergrößert, wodurch der am Eingang des Phasenkontrast-Differenzverstärkers 14 gegebene Phasengradient beim Durchgang durch den Phasenkontrast-Differenzverstärker 14 verstärkt wird. Es erfolgt also quasi eine Phasenverschiebung aufgrund unterschiedlicher Weglängen durch das brechende Material.

Basierend auf diesem Effekt lässt sich nun ein Phasenkontrast-Differenzverstärker 14 realisieren, welcher aus zwei Brechungsgittern $G_A$ und $G_B$ aufgebaut ist, die eine regelmäßige Struktur aus brechendem Material aufweisen, durch welche quasi brechende Prismen realisiert sind, die abwechselnd in Richtung einer x-Achse und entgegen der Richtung der x-Achse brechen, wenn Wellenfronten unter einem bestimmten Winkel auf die Brechungsgitter $G_A$ und $G_B$ auftreffen, wenn also ein Strahl nicht senkrecht zur Oberfläche eintritt.

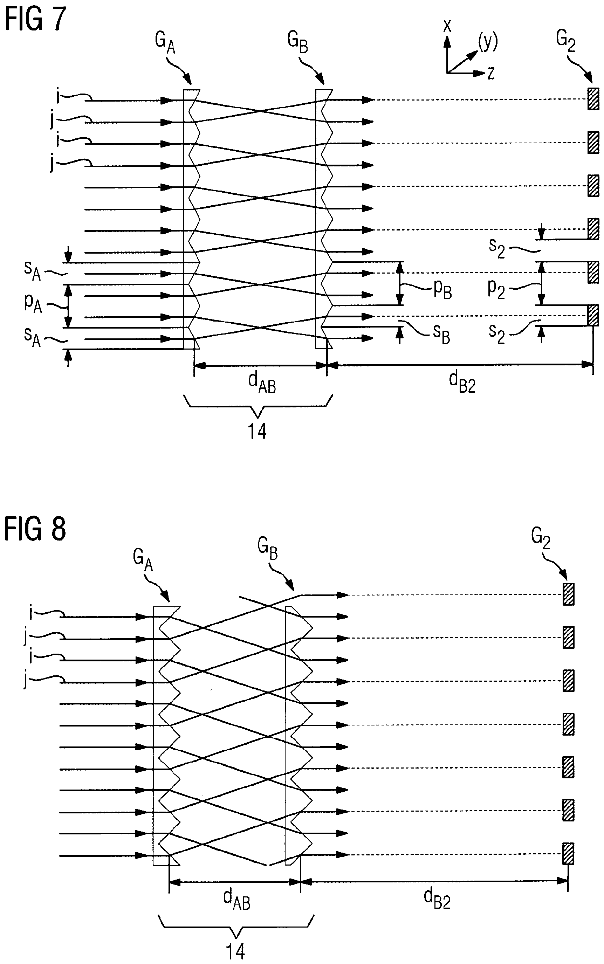

Entsprechende regelmäßige Strukturen sind in den Darstellungen FIG 6 bis FIG 9 angedeutet. Die Abbildungen zeigen dabei in Richtung der x-Achse unterschiedliche stark komprimierte Skizzen, d.h., dass die x-Achse müsste eigentlich stark gegenüber der Darstellung gestreckt werden. In den Darstellungen sind dabei unterschiedliche Prismenstärken, also unterschiedliche Gradienten der geneigten Kanten, dargestellt, wobei die Prismenstärke eines Brechungsgitters vorzugsweise stets so zu bemessen ist, dass das streifenförmige Interferenzmuster durch den Phasenkontrast-Differenzverstärker 14 um eine ganzzahlige Streifenzahl N oder ein ganzzahliges Vielfaches N der Streifenbreite verschoben wird.

Das Verschieben bedingt dabei auch eine Phasenverschiebung um den Gradient entlang von N-Streifen, so dass am Brechungsgitter $G_B$ austretende benachbarte Teilstrahlen i, j das 2N-fache der Phasenverschiebung gegenüber der Phasenverschiebung dieser Teilstrahlen am Eingang des Phasenkontrast-Differenzverstärkers 14 aufweisen. Hierbei handelt es sich um einen weiteren Effekt, bei dem ein Phasensprung aufgrund der Verschiebung des Interferenzmusters gegeben ist.

Dieser zusätzliche Effekt wird jedoch durch einen weiteren Effekt etwa gleicher Stärke kompensiert, der anhand der Darstellung FIG 5 nachvollziehbar ist. Vergleicht man die Strahlengänge b und c sowie f und g, so erkennt man, dass die elektromagnetische Strahlung je nach Strahlengang unterschiedliche Wegstrecken entlang einer x-Achse senkrecht zur z-Achse zu überwinden hat, was die Phase der elektromagnetischen Strahlung ebenfalls beeinflusst. Durch entsprechende Rechnungen lässt sich zeigen, dass alle drei Effekte in etwa dieselbe Stärke haben, so dass sich quasi zwei Effekte gegenseitig aufheben.

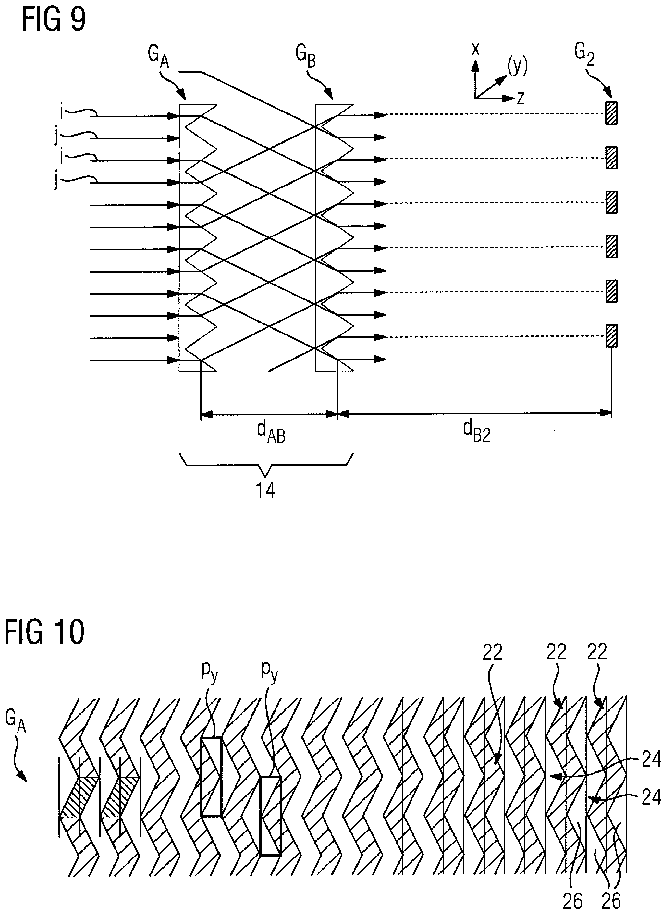

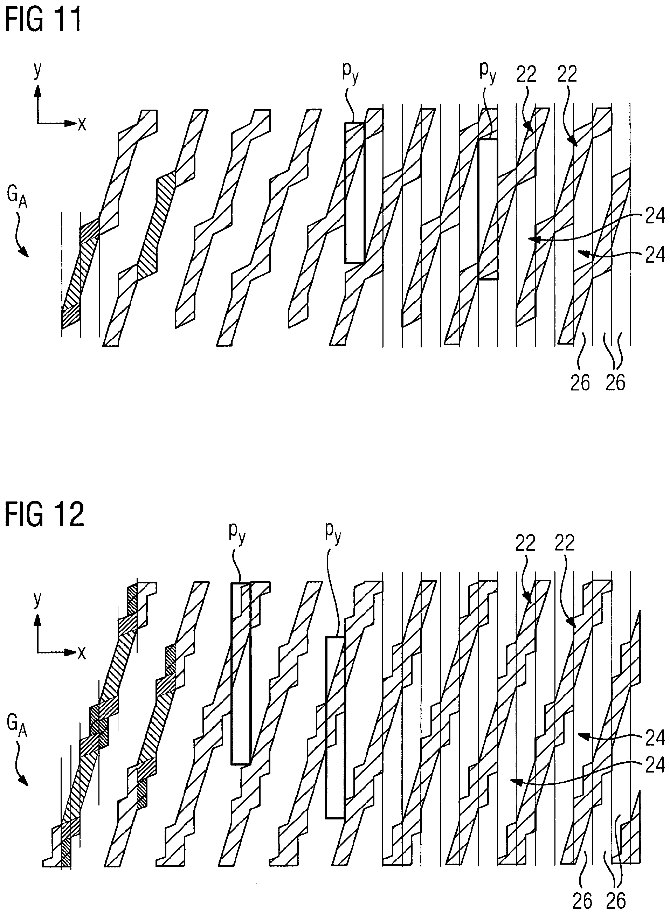

Die benötigten Strukturen für die Brechungsgitter $G_A$ und $G_B$ lassen sich beispielsweise durch schräg belichtete Photolithografie realisieren und hierfür geeignete Layouts sind in den Darstellungen FIG 10 bis FIG 12 wiedergegeben. Abgebildet ist hierbei jeweils die Stirnfläche oder Gitterebene eines Brechungsgitters $G_A$ oder $G_B$ quer zur z-Achse, welche in Richtung der x-Achse und in Richtung einer y-Achse senkrecht dazu erstreckt.

Die Layouts zeigen dabei unterschiedlich ausgestaltete Brechungsstege 22, durch welche auftreffende elektromagnetische Strahlung eine Phasenverschiebung erfährt, wobei die Brechungsstege 22 durch Zwischenräume 24 voneinander getrennt sind, durch welche elektromagnetische Strahlung im Wesentlichen ohne eine Phasenverschiebung hindurchtreten kann. Mit Hilfe der Brechungsstege 22 ist dann ein Brechungsgitter $G_A$ oder $G_B$ realisiert, bei dem Röntgenstrahlung in aufeinanderfolgenden Brechungsstreifen 26 abwechselnd, also alternierend, nach links und nach rechts gebrochen wird, also in Richtung der x-Achse bzw. entgegen der x-Achse.

Die Erfindung ist nicht auf das vorstehend beschriebene Ausführungsbeispiel beschränkt. Vielmehr können auch andere Varianten der Erfindung von dem Fachmann hieraus abgeleitet werden, ohne den Gegenstand der Erfindung zu verlassen. Insbesondere sind ferner alle im Zusammenhang mit dem Ausführungsbeispiel beschriebenen Einzelmerkmale auch auf andere Weise miteinander kombinierbar, ohne den Gegenstand der Erfindung zu verlassen.

**Patentansprüche**

1.  Phasenkontrast-Röntgenbildgebungsvorrichtung (2), insbesondere für den Medizinbereich, umfassend eine Röntgenstrahlungsquelle (6) zur Generierung eines Röntgenstrahlungsfeldes und einen Röntgendetektor (8) mit einer eindimensionalen oder zweidimensionalen Anordnung von Pixeln (10), wobei zwischen der Röntgenstrahlungsquelle (6) und dem Röntgendetektor (10) ein Phasenkontrast-Differenzverstärker (14) positioniert ist, mit dessen Hilfe im Betrieb räumliche Phasenunterschiede im Röntgenstrahlungsfeld verstärkt werden, wobei der Phasenkontrast-Differenzverstärker (14) zwei Brechungsgitter ($G_A$,$G_B$) umfasst, die in einer Strahlungseinfallrichtung (z) gesehen nacheinander angeordnet sind, **dadurch gekennzeichnet, dass** ein jedes Brechungsgitter ($G_A$,$G_B$)

    - eine im Wesentlichen quer zur Strahlungseinfallrichtung (z) auszurichtende Transversalfläche aufweist, die durch eine x-Achse (x) und eine hierzu senkrechte y-Achse (y) aufgespannt ist und
    - eine Vielzahl von Brechungsstegen (22) aus einem optisch vergleichsweise dünnen Basismaterial umfasst, die alternierend mit optisch dichteren Zwischenräumen (24) angeordnet sind, wobei die Brechungsstege (22) derart ausgebildet sind, dass diese die Transversalfläche in jeweils in y-Richtung (y) langgestreckte Brechungsstreifen (26) gliedern, die in x-Richtung (x) parallel nebeneinander aufgereiht sind und wobei benachbarte Brechungsstreifen (26) sich dadurch unterscheiden, dass sie hinsichtlich der Brechungseigenschaften des im Bereich des Brechungsstreifens (26) jeweils angeordneten Gittermaterials stets auf verschiedene Fokusse ausgerichtet sind oder in verschiedene Richtungen brechen.

2.  Phasenkontrast-Röntgenbildgebungsvorrichtung (2) nach Anspruch 1,
    **dadurch gekennzeichnet, dass** der Phasenkontrast-Differenzverstärker (14) derart ausgestaltet ist, dass unbeeinflusste Röntgenstrahlung (18) eine einheitliche Phasenverschiebung durch den Phasenkontrast-Differenzverstärker (14) erfährt unabhängig von der Eintrittsposition der unbeeinflussten Röntgenstrahlung (18) am Phasenkontrast-Differenzverstärker (14).

3.  Phasenkontrast-Röntgenbildgebungsvorrichtung (2) nach Anspruch 1 oder 2,
    **dadurch gekennzeichnet, dass** der Phasenkontrast-Differenzverstärker (14) zwei gleiche Brechungsgitter ($G_A$,$G_B$) aufweist.

4.  Phasenkontrast-Röntgenbildgebungsvorrichtung (2) nach einem der Ansprüche 1 bis 3,
    **dadurch gekennzeichnet, dass** die Brechungsstege (22) derart ausgebildet sind, dass sie innerhalb der Transversalfläche zumindest abschnittsweise diagonal verlaufen, wobei sich die Seitenflächen mindestens eines Brechungsstegs (22), die diesen Brechungssteg (22) in x-Richtung (x) begrenzen, jeweils über mehrere Brechungsstreifen (26) erstrecken.

5.  Phasenkontrast-Röntgenbildgebungsvorrichtung (2) nach einem der Ansprüche 1 bis 4,
    **dadurch gekennzeichnet, dass** die Brechungsstege (22) jeweils nach Art von in y-Richtung (y) geneigten schiefen Prismen geformt sind, deren Grundfläche und Deckfläche in den zur Transversalfläche parallelen Stirnflächen des Brechungsgitters ($G_A$,$G_B$) liegen.

6.  Phasenkontrast-Röntgenbildgebungsvorrichtung (2) nach Anspruch 5,
    **dadurch gekennzeichnet, dass** die Brechungsstege (22) derart angeordnet sind, dass in jedem Brechungsstreifen (26) eine sich in y-Richtung (y) mit einer y-Periodenlänge ($p_y$) wiederholende Materialstruktur ergibt, und wobei die Brechungsstege (22) derart in y-Richtung (y) geneigt sind, dass eine zu der Grundfläche entgegengesetzte Deckfläche eines jeden Brechungsstegs (22) gegenüber der Grundfläche um eine ganze Anzahl von Periodenlängen, insbesondere genau eine Periodenlänge versetzt ist.

7.  Phasenkontrast-Röntgenbildgebungsvorrichtung (2) nach einem der Ansprüche 1 bis 6,
    **dadurch gekennzeichnet, dass** jeder Brechungssteg (22) durch jeweils zwei Seitenflächen an die zwischen den Brechungsstegen (22) angeordneten Zwischenräume (24) angrenzt, wobei die Seitenflächen alternierend aus aktiven Teilflächen mit vergleichsweise starker Brechungswirkung in x-Richtung (x) und passiven Teilflächen mit geringer oder verschwindender Brechungswirkung in x-Richtung (x) zusammengesetzt sind.

8.  Phasenkontrast-Röntgenbildgebungsvorrichtung (2) nach Anspruch 7,
    **dadurch gekennzeichnet, dass** sich jede aktive oder passive Teilfläche in x-Richtung über eine ganze Anzahl von Brechungsstreifen (26) erstreckt.

9.  Phasenkontrast-Röntgenbildgebungsvorrichtung (2) nach einem der Ansprüche 1 bis 8,
    **dadurch gekennzeichnet, dass** zwischen der Röntgenstrahlungsquelle (6) und dem Phasenkontrast-Differenzverstärker (14) eine Objektaufnahme (12) zur Aufnahme eines Untersuchungsobjektes angeordnet ist.

10. Phasenkontrast-Röntgenbildgebungsvorrichtung (2) nach einem der Ansprüche 1 bis 9, **dadurch gekennzeichnet, dass** zwischen dem Phasenkontrast-Differenzverstärker (14) und dem Röntgendetektor (8) ein Analysegitter (G₂) angeordnet ist.

11. Phasenkontrast-Röntgenbildgebungsvorrichtung (2) nach einem der Ansprüche 1 bis 10, **dadurch gekennzeichnet, dass** zwischen der Röntgenstrahlungsquelle (6) und dem Phasenkontrast-Differenzverstärker (14) ein Kohärenzgitter (G₀) angeordnet ist.

12. Phasenkontrast-Röntgenbildgebungsvorrichtung (2) nach einem der Ansprüche 1 bis 11, **dadurch gekennzeichnet, dass** zwischen dem Phasenkontrast-Differenzverstärker (14) und dem Röntgendetektor (8) ein zusätzliches Brechungsgitter (G_L) angeordnet ist.

**Claims**

1. Phase-contrast x-ray imaging device (2), in particular for the medical field, comprising an x-radiation source (6) for generating an x-radiation field and an x-ray detector (8) having a one-dimensional or two-dimensional arrangement of pixels (10), wherein a phase-contrast differential amplifier (14), with the aid of which spatial phase differences in the x-radiation field are amplified during operation, is positioned between the x-radiation source (6) and the x-ray detector (10), wherein the phase-contrast differential amplifier (14) comprises two diffraction gratings (G_A,G_B) which, when viewed in a radiation incidence direction (z), are arranged one behind the other, **characterised in that** each diffraction grating (G_A,G_B)

   - has a transverse surface which is to be aligned substantially at right angles to the radiation incidence direction (z) and which is spanned by an x-axis (x) and a y-axis (y) perpendicular thereto, and
   - comprises a plurality of diffraction ridges (22) made of an optically comparatively thin base material which are arranged alternating with optically denser interspaces (24), wherein the diffraction ridges (22) are embodied in such a way that they subdivide the transverse surface into elongate diffraction strips (26) which extend in each case in the y-direction (y) and which are arranged next to one another in parallel rows in the x-direction (x) and wherein adjacent diffraction strips (26) are different from one another **in**

**that** they are always aligned to different focuses in terms of the diffraction properties of the grating material arranged in each case in the vicinity of said diffraction strip (26) or diffract in different directions.

2. Phase-contrast x-ray imaging device (2) according to claim 1, **characterised in that** the phase-contrast differential amplifier (14) is embodied in such a way that unaffected x-radiation (18) experiences a uniform phase shift due to the phase-contrast differential amplifier (14) irrespective of the entry position of the unaffected x-radiation (18) at the phase-contrast differential amplifier (14).

3. Phase-contrast x-ray imaging device (2) according to one of claims 1 or 2, **characterised in that** the phase-contrast differential amplifier (14) has two identical diffraction gratings (G_A,G_B).

4. Phase-contrast x-ray imaging device (2) according to one of claims 1 to 3, **characterised in that** the diffraction ridges (22) are embodied in such a way that they extend diagonally at least in sections within the transverse surface, wherein the lateral faces of at least one diffraction ridge (22) which delimit said diffraction ridge (22) in the x-direction (x) in each case extend across a plurality of diffraction strips (26).

5. Phase-contrast x-ray imaging device (2) according to one of claims 1 to 4, **characterised in that** the diffraction ridges (22) are in each case implemented in the manner of oblique prisms inclined in the y-direction (y), the base surface and top surface of which lie in the end faces of the diffraction grating (G_A,G_B) that are parallel to the transverse surface.

6. Phase-contrast x-ray imaging device (2) according to claim 5, **characterised in that** the diffraction ridges (22) are arranged in such a way that in each diffraction strip (26) there results a material structure repeating itself with a y-period length (p_y) in the y-direction (y), and wherein the diffraction ridges (22) are inclined in the y-direction (y) in such a way that a top surface of each diffraction ridge (22) opposite the base surface is offset with respect to the base surface by a whole number of period lengths, in particular by precisely one period length.

7. Phase-contrast x-ray imaging device (2) according to one of claims 1 to 6, **characterised in that** each diffraction ridge (22) adjoins the interspaces (24) arranged between the diffraction ridges (22) by means of two lateral faces in each case, wherein the lateral faces are composed of active subareas having a comparatively strong diffraction effect in the x-direction (x) alternating with passive subareas having a small or vanishing diffraction effect in the x-direction (x).

8. Phase-contrast x-ray imaging device (2) according to claim 7,
   **characterised in that** each active or passive subarea extends across a whole number of diffraction strips (26) in the x-direction.

9. Phase-contrast x-ray imaging device (2) according to one of claims 1 to 8,
   **characterised in that** an object support (12) for accommodating an examination object is arranged between the x-radiation source (6) and the phase-contrast differential amplifier (14).

10. Phase-contrast x-ray imaging device (2) according to one of claims 1 to 9,
    **characterised in that** an analysis grating ($G_2$) is arranged between the phase-contrast differential amplifier (14) and the x-ray detector (8).

11. Phase-contrast x-ray imaging device (2) according to one of claims 1 to 10,
    **characterised in that** a coherence grating ($G_0$) is arranged between the x-radiation source (6) and the phase-contrast differential amplifier (14).

12. Phase-contrast x-ray imaging device (2) according to one of claims 1 to 11,
    **characterised in that** an additional diffraction grating ($G_L$) is arranged between the phase-contrast differential amplifier (14) and the x-ray detector (8).

**Revendications**

1. Dispositif (2) d'imagerie radiographique par contraste de phase, notamment pour le domaine de la médecine, comprenant une source (6) de rayons X pour créer un champ de rayonnement X et un détecteur (8) de rayons X ayant un agencement monodimensionnel ou bidimensionnel de pixels (10), dans lequel il est placé, entre la source (6) de rayonnement X et le détecteur (10) de rayons X, un renforçateur (14) de différence de contraste de phase, à l'aide duquel, en fonctionnement, des différences de phase spatiale dans le champ de rayonnement X sont renforcées, le renforçateur (14) de différence de contraste de phase comprenant deux réseaux ($G_A$, $G_B$) de diffraction qui, considérés dans un sens (z) d'incidence du rayonnement, sont disposés l'un après l'autre, **caractérisé en ce que** chaque réseau ($G_A$, $G_B$) de diffraction

- a une surface transversale orientée sensiblement transversalement au sens d'incidence du rayonnement et passant par un axe x (x) et par un axe y (y), qui lui est perpendiculaire et
- comprend une pluralité de barrettes (22) de diffraction en un matériau de base optiquement relativement mince, qui sont disposées en alternance avec des espaces (24) intermédiaires optiquement plus denses, les barrettes (22) de diffraction étant constituées de manière à diviser les surfaces transversales en des bandes (26) de diffraction s'étendant en longueur dans la direction y (y), qui sont rangées parallèlement les unes à côté des autres dans la direction x (x), et dans lequel des bandes (26) de diffraction voisines se distinguent par le fait qu'elles sont dirigées, en ce qui concerne les propriétés de diffraction du matériau du réseau disposé dans la région de la bande (26) de diffraction, toujours sur des foyers différents ou diffractent dans des directions différentes.

2. Dispositif (2) d'imagerie radiographique par contraste de phase suivant la revendication 1,
   **caractérisé en ce que** le renforçateur (14) de différence de contraste de phase est constitué de manière à ce qu'un rayonnement (18) X non-influencé subisse un déphasage unitaire par le renforçateur (14) de différence de contraste de phase, indépendamment de la position d'entrée du rayonnement (18) X non-influencé, dans le renforçateur (14) de différence de contraste de phase.

3. Dispositif (2) d'imagerie radiographique par contraste de phase suivant la revendication 1 ou 2,
   **caractérisé en ce que** le renforçateur (14) de différence de contraste de phase a deux réseaux ($G_A$, $G_B$) de diffraction.

4. Dispositif (2) d'imagerie radiographique par contraste de phase suivant l'une des revendications 1 à 3,
   **caractérisé en ce que** les barrettes (22) de diffraction sont constituées, de manière à s'étendre en diagonale, au moins par tronçon, au sein de la surface transversale, les surfaces latérales d'au moins une barrette (22) de réfraction, qui délimite cette barrette (22) de réfraction dans la direction x (x), s'étendant chacune sur plusieurs bandes (26) de diffraction.

5. Dispositif (2) d'imagerie radiographique par contraste de phase suivant l'une des revendications 1 à 4,
   **caractérisé en ce que** les barrettes (22) de diffraction sont formées chacune à la manière de prismes gauches inclinés dans la direction y (y), dont la surface de base et la surface de sommet se trouvent dans les surfaces frontales, parallèles à la surface transversale, du réseau ($G_A$, $G_B$) de diffraction.

**6.** Dispositif (2) d'imagerie radiographique par suivant la revendication 5,
**caractérisé en ce que** les barrettes (22) de diffraction sont disposées, de manière à donner, dans chaque bande (26) de diffraction, une structure de matériau se répétant dans la direction y (y) à une période y ($p_y$), et les barrettes (22) de diffraction sont inclinées dans la direction y (y), de manière à ce qu'une surface de sommet opposée à la surface de base de chaque barrette (22) de diffraction soit décalée par rapport à la surface de base, notamment d'un nombre entier de périodes, notamment exactement d'une période.

**7.** Dispositif (2) d'imagerie radiographique par contraste de phase suivant l'une des revendications 1 à 6,
**caractérisé en ce que** chaque barrette (22) de diffraction est délimitée par respectivement deux surfaces latérales sur les espaces (14) intermédiaires disposés entre les barrettes (22) de diffraction, les surfaces latérales étant composées en alternance de sous-surfaces actives à effet de diffraction relativement fort dans la direction x (x) et de sous-surfaces passives à effet de diffraction petit ou disparaissant dans la direction x (x).

**8.** Dispositif (2) d'imagerie radiographique par contraste de phase suivant la revendication 7,
**caractérisé en ce que** chaque sous-surface active ou passive s'étend dans la direction x sur un nombre entier de bandes (26) de diffraction.

**9.** Dispositif (2) d'imagerie radiographique par contraste de phase suivant l'une des revendications 1 à 8,
**caractérisé en ce qu'**un logement (12) objet, de réception d'un objet à étudier, est disposé entre la source (6) de rayonnement X et le renforçateur (14) de différence de contraste de phase.

**10.** Dispositif (2) d'imagerie radiographique par contraste de phase suivant l'une des revendications 1 à 9,
**caractérisé en ce qu'**un réseau ($G_2$) d'analyse est disposé entre le renforçateur (14) de différence de contraste de phase et le détecteur (8) de rayons X.

**11.** Dispositif (2) d'imagerie radiographique par contraste de phase suivant l'une des revendications 1 à 10,
**caractérisé en ce qu'**un réseau ($G_0$) de cohérence est disposé entre la source (6) de rayonnement X et le renforçateur (14) de différence de contraste de phase.

**12.** Dispositif (2) d'imagerie radiographique par contraste de phase suivant l'une des revendications 1 à 11,
**caractérisé en ce qu'**un réseau ($G_L$) de diffraction supplémentaire est disposé entre le renforçateur (14) de différence de contraste de phase et le détecteur (8) de rayons X.

FIG 1

FIG 2

## FIG 3

## FIG 4

FIG 5

FIG 6

## FIG 7

## FIG 8

## FIG 9

## FIG 10

FIG 11

FIG 12

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2013004574 A1
- US 20120041679 A1

- WO 2013160153 A1
- WO 2013160153 A

**In der Beschreibung aufgeführte Nicht-Patentliteratur**

- **T. WEITKAMP.** X-ray phase imaging with a grating interferometer. *OPTICS EXPRESS,* 08. August 2005, vol. 13 (16